# EUROPEAN PATENT SPECIFICATION

(11) **EP 1 283 888 B1**
(45) Date of publication and mention of the grant of the patent: **04.06.2008**
(21) Application number: 01934630.3
(22) Date of filing: 23.05.2001
(51) Int. Cl.: C12N 15/54, C12N 9/10, C12N 1/21, C12P 19/18, C08B 37/00, A23C 9/123, C12R 1/225

(54) **FRUCTOSYLTRANSFERASES (INULOSUCRASE AND LEVANSUCRASE) FROM LACTOBACILLUS REUTERI**
FRUKTOSYLTRANSFERASE (INULOSUCRASE UND LEVANSUCRASE) VON LACTOBACILLUS REUTERI
FRUCTOSYLTRANSFERASES (INULOSUCRASE ET LEVANSUCRASE) DERIVEES DE LACTOBACILLUS REUTERI

(30) Priority: 25.05.2000 EP 00201872; 09.01.2001 EP 01200049
(43) Date of publication of application: 19.02.2003
(73) Proprietor: Nederlandse Organisatie voor Toegepast-Natuurwetenschappelijk Onderzoek TNO, 2628 VK Delft (NL)
(72) Inventor: VAN GEEL-SCHUTTEN, Gerritdina, Hendrika, NL-3971 XG Driebergen (NL); RAHAOUI, Hakim, NL-3813 VS Amersfoort (NL); DIJKHUIZEN, Lubbert, NL-9742 RB Zuidlaren (NL); VAN HIJUM, Sacha, Adrianus, Fokke, Taco, NL-9716 BH Groningen (NL)
(74) Representative: van Westenbrugge, Andries
(86) International application number: PCT/NL2001/000392
(87) International publication number: WO 2001/090319

(56) References cited:
- VAN GEEL-SCHUTTEN, G. H. ET AL: "Exopolysaccharide production by Lactobacillus reuteri, involving sucrase type of enzymes" MEDED. - FAC. LANDBOUWKD. TOEGEPASTE BIOL. WET. (UNIV. GENT), vol. 65, no. 3a, 2000, pages 197-201, XP000971140
- VAN GEEL-SCHUTTEN G H ET AL: "Biochemical and structural characterization of the glucan and fructan exopolysaccharides synthesized by the Lactobacillus reuteri wild-type strain and by mutant strains." APPLIED AND ENVIRONMENTAL MICROBIOLOGY, vol. 65, no. 7, July 1999 (1999-07), pages 3008-3014, XP002154732 ISSN: 0099-2240 cited in the application
- SHIROZA T ET AL: "SEQUENCE ANALYSIS OF THE STREPTOCOCCUS-MUTANS FRUCTOSYLTRANSFERASE GENE AND FLANKING REGIONS" JOURNAL OF BACTERIOLOGY, vol. 170, no. 2, 1988, pages 810-816, XP001002139 ISSN: 0021-9193 cited in the application
- VAN GEEL-SCHUTTEN G H : "Exopolysaccharide synthesis by Lactobacillus reuteri: Molecular characterization of a fructosyltransferase and a glucansucrase" 16 June 2000 (2000-06-16) , PHD THESIS, RIJKSUNIVERSITEIT GRONINGEN XP002191551 chapters 3 and 4

## Description

The present invention is in the field of enzymatic production of biomolecules. The invention is particularly concerned with novel fructosyltransferases derived from lactobacilli and with a process for recombinant production of the enzymes and for the production of useful inulins and fructo-oligosaccharides from sucrose.

### Background of the invention

Lactic acid bacteria (LAB) play an important role in the fermentative production of food and feed. Traditionally, these bacteria have been used for the production of for instance wine, beer, bread, cheese and yoghurt, and for the preservation of food and feed, e.g. olives, pickles, sausages, sauerkraut and silage. Because of these traditional applications, lactic acid bacteria are food-grade micro-organisms that posses the Generally Recognised As Safe (GRAS) status. Due to the different products which are formed during fermentation with lactic acid bacteria, these bacteria contribute positively to the taste, smell and preservation of the final product. The group of lactic acid bacteria encloses several genera such as *Lactobacillus, Leuconostoc, Pediococcus, Streptococcus,* etc.

In recent years also the health promoting properties of lactic acid bacteria have received much attention. They produce an abundant variety of exopolysaccharides (EPS's). These polysaccharides are thought to contribute to human health by acting as prebiotic substrates, nutraceuticals, cholesterol lowering agents or immunomodulants.

To date high molecular weight polysaccharides produced by plants (such as cellulose, starch and pectin), seaweeds (such as alginate and carrageenan) and bacteria (such as alginate, gellan and xanthan) are used in several industrial applications as viscosifying, stabilising, emulsifying, gelling or water binding agents. Although all these polysaccharides are used as food additives, they originate from organisms not having the GRAS status. Thus they are less desirable than the exopolysaccharides of micro-organisms, such as lactic acid bacteria, wich have the GRAS status.

The exopolysaccharides produced by LAB can be divided in two groups, heteropolysaccharides and homopolysaccharides; these are synthesized by totally different mechanisms. The former consist of repeating units in which residues of different types of sugars are present and the latter consist of one type of monosaccharide. The synthesis of heteropolysaccharides by lactic acid bacteria, including lactobacilli, has been studied extensively in recent years. Considerably less information is available on the synthesis of homopolysaccharides from lactobacilli, although some studies have been performed. Homopolysaccharides with fructose as the constituent sugar can be divided into two groups, inulins and levans. Inulins consist of 2,1-linked β-fructofuranoside residues, whereas levans consist of 2,6-linked β-fructofuranoside residues. Both can be linear or branched. The size of bacterial levans can vary from 20 kDa up to several MDa. There is limited information on the synthesis of levans. In most detail this synthesis has been studied in *Zymomonas mobilis* and in *Bacillus* species. Within lactic acid bacteria, fructosyltransferases have only been studied in streptococci. So far no fructosyltransferases have been reported in lactobacilli.

In a recent report the *Lactobacillus reuteri* strain LB 121 was found to produce both a glucan and a fructan when grown on sucrose, but only a fructan when grown on raffinose (van Geel-Schutten, G.H. et al., Appl. Microbiol. Biotechnol. (1998) 50, 697-703). In another report the glucan and fructan were characterised by their molecular weights (of 3,500 and 150 kDa respectively) and the glucan was reported to be highly branched with a unique structure consisting of a terminal, 4-substituted, 6-substituted, and 4,6-disubstituted α-glucose in a molar ratio 1.1 : 2.7 : 1.5 : 1.0 (van Geel-Schutten, G.H. et al., Appl. Environ. Microbiol. (1999) 65, 3008-3014). The fructan was identified as a linear (2→6)-β-D-fructofuranan (also called a levan). This was the first example of fructan synthesis by a *Lactobacillus* species.

### Summary of the invention

Two novel genes encoding enzymes having fructosyltransferase activity have now been found in *Lactobacillus reuteri,* and their amino acid sequences have been determined. These are the first two enzymes identified in a *Lactobacillus* species capable of producing a fructan. One of the enzymes is an inulosucrase which produces a high molecular weight (>10⁷ Da) fructan containing β(2-1) linked fructosyl units and fructo-oligosaccharides, while the other is a levansucrase which produces a fructan containing β(2-6) linked fructosyl units. The invention pertains to the inulosucrase enzyme, to DNA encoding it, to recombinant cells containing such DNA and to their use in producing carbohydrates, as defined in the appending claims.

### Description of the invention

It was found according to the invention that one of the novel fructosyltransferases (FTFA; an inulosucrase) produces a high molecular weight inulin with β(2-1) linked fructosyl units and fructo-oligosaccharides. The fructo-oligosaccharides synthesis was also observed in certain *Lactobacillus* strains, in particular in certain strains of *Lactobacillus reuteri.* However, the inulin has not been found in *Lactobacillus reuteri* culture supernatants, but only in extracts of *E. coli* cells expressing the above-mentioned fructosyltransferase. This inulosucrase consists of either 798 amino acids (2394 nucleotides) or 789 amino acids (2367 nucleotides) depending on the potential start codon used. The molecular weight (MW) deduced of the amino acid sequence of the latter form is 86 kDa and its isoelectric point is 4.51, at pH 7.

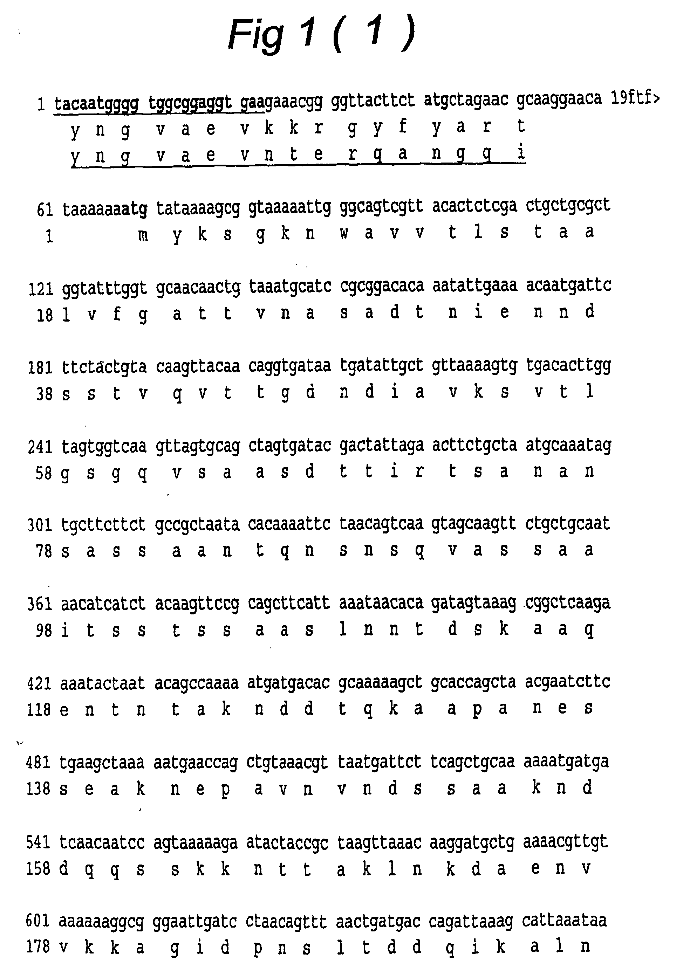

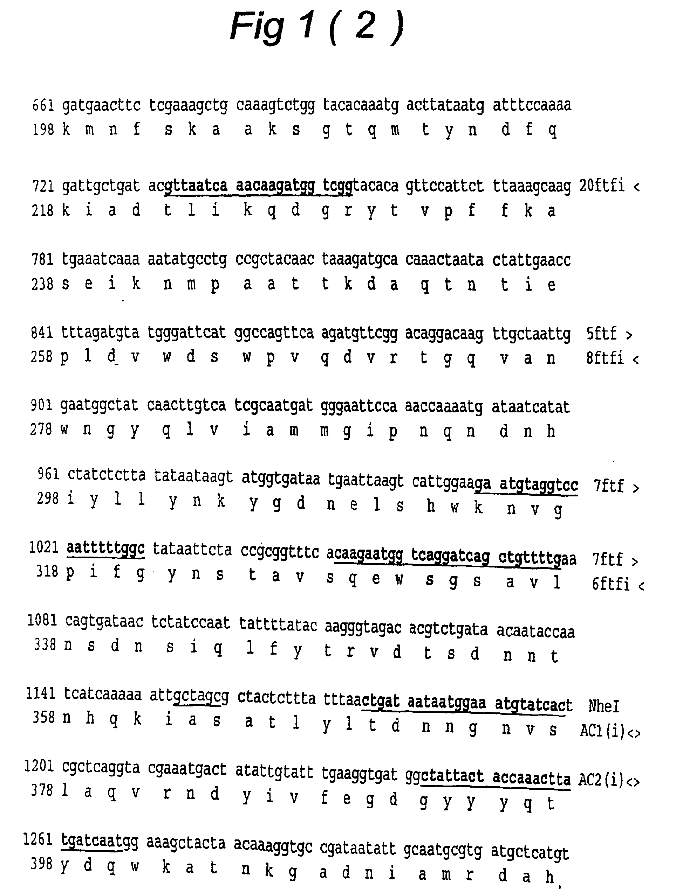

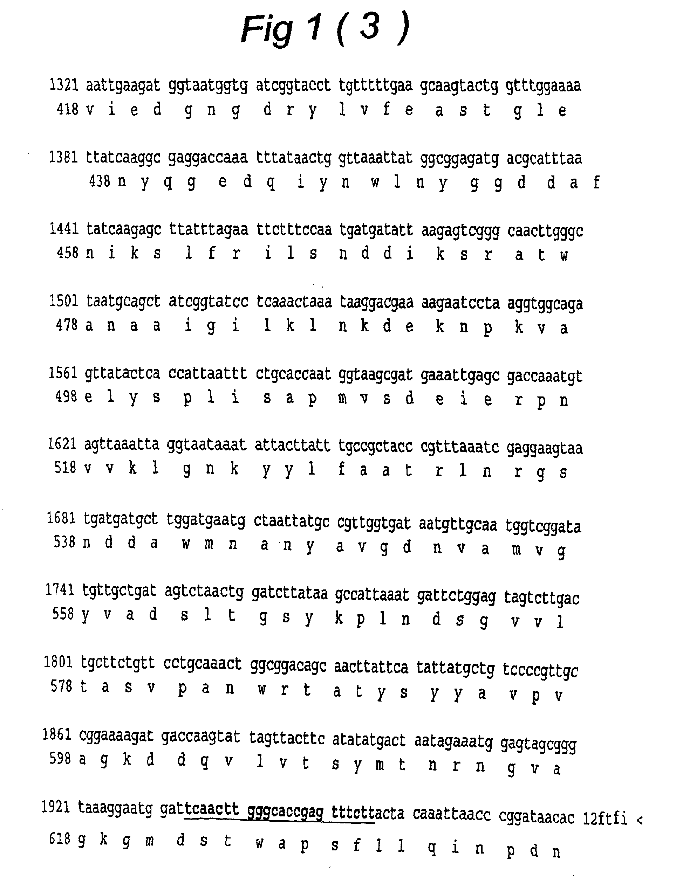

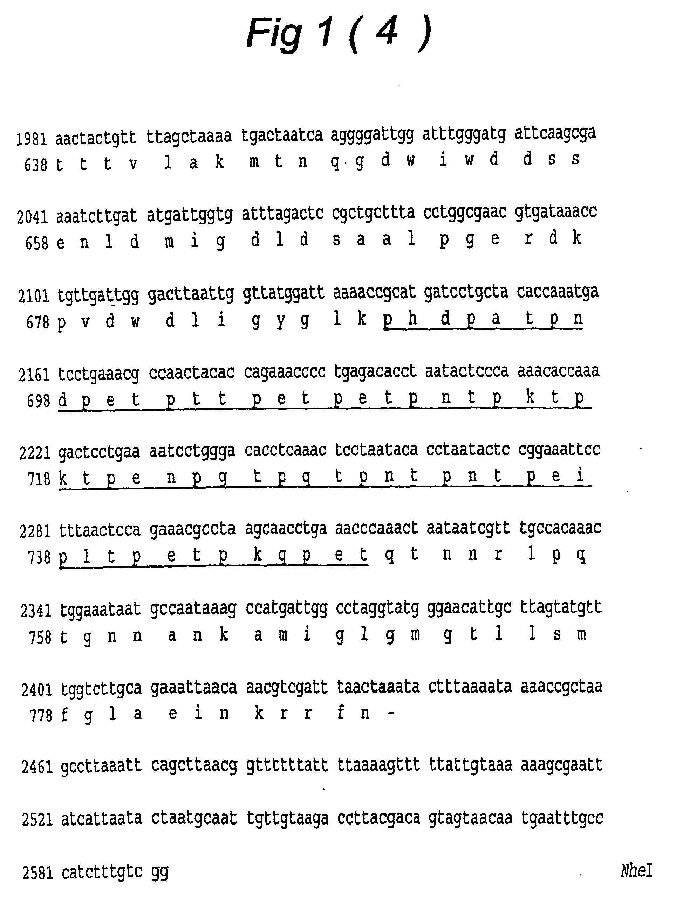

The amino acid sequence of the inulosucrase is shown in SEQ ID No. 1 (figure 1, amino acid residues 1-789). As mentioned above, the nucleotide sequence contains two putative start codons leading to either a 2394 (see SEQ ID No. 3) or 2367 (see SEQ ID No. 2) nucleotide form of the inulosucrase. Both putative start codons are preceded by a putative ribosome binding site, GGGG (located 12 base pairs upstream its start codon) or AGGA (located 14 base pairs upstream its start codon), respectively (see figure 1 and SEQ ID No. 4).

The present invention covers a protein having inulosucrase activity with an amino acid identity of at least 65%, preferably at least 75%, and more preferably at least 85%, compared to the amino acid sequence of SEQ ID No. 1. The invention also covers a part of a protein with at least 15 contiguous amino acids which are identical to the corresponding part of the amino acid sequence of SEQ ID No. 1.

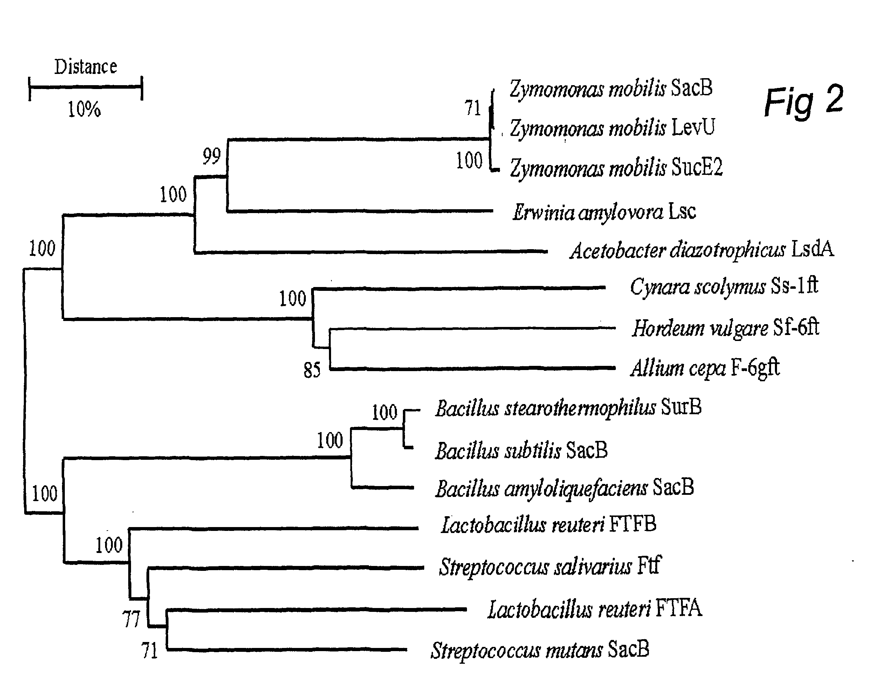

Fructosyltransferases have been found in several bacteria such as *Zymomonas mobilis, Erwinia amylovora, Acetobacter amylovora, Bacillus polymyxa, Bacillus amyloliquefaciens, Bacillus stearothermophilus,* and *Bacillus subtilis.* In lactic acid bacteria this type of enzyme previously has only been found in some streptococci. Most bacterial fructosyltransferases have a molecular mass of 50-100 kDa (with the exception of the fructosyltransferase found in *Streptococcus salivarius* which has a molecular mass of 140 kDa). Amino acid sequence alignment revealed that the novel inulosucrase of lactobacilli has high homology with fructosyltransferases originating from Gram positive bacteria, in particular with *Streptococcus* enzymes. The highest homology (figure 2) was found with the SacB enzyme of *Streptococcus mutans* Ingbritt A (62% identity within 539 amino acids).

Certain putative functions based on the alignment and site-directed mutagenesis studies can be ascribed to several amino acids of the novel inulosucrase. Asp-263, Glu-330, Asp-415, Glu-431, Asp-511, Glu-514, Arg-532 and/or Asp-551 of the amino acid sequence of SEQ ID No. 1 are identified as putative catalytic residues. Noteworthy, a hydrophobicity plot according to Kyte and Doolittle (1982) J. Mol. Biol. 157, 105-132 suggests that the novel inulosucrase contains a putative signal sequence according to the Von Heijne rule. The putative signal peptidase site is located between Gly at position 21 and Ala at position 22. Furthermore, it is striking that the C-terminal amino acid sequence of the novel inulosucrase contains a putative cell wall anchor amino acid signal LPXTG (SEQ ID No. 5) and a 20-fold repeat of the motif PXX (see figure 1), where P is proline and X is any other amino acid. In 15 out of 20 repeats, however, the motif is PXT. This motif has so far not been reported in proteins of prokaryotic and eukaryotic origin.

A nucleotide sequence encoding any of the above mentioned proteins, mutants, variants or parts thereof is also a subject of the invention. Furthermore, the nucleic acid sequences corresponding to expression-regulating regions (promoters, enhancers, terminators) of at least 30 contiguous nucleic acids contained in the nucleic acid sequence (1)-(67) or (2438)-(2592) of SEQ ID No. 4 (see also figure 1) can be used for homologous or heterologous expression of genes. Such expression-regulating sequences are operationally linked to a polypeptide-encoding nucleic acid sequence such as the genes of the fructosyltransferase according to the invention. A nucleic acid construct comprising the nucleotide sequence operationally linked to an expression-regulating nucleic acid sequence is also covered by the invention.

A recombinant host cell, such as a mammalian (with the exception of human), plant, animal, fungal or bacterial cell, containing one or more copies of the nucleic acid construct mentioned above is an additional subject of the invention. The inulosucrase gene (starting at nucleotide 41) has been cloned in an *E. coli* expression vector under the control of an *ara* promoter in *E. coli* Top10. *E. coli* Top10 cells expressing the recombinant inulosucrase hydrolysed sucrose and synthesized fructan material. SDS-PAGE of arabinose induced *E. coli* Top10 cell extracts suggested that the recombinant inulosucrase has a molecular weight of 80-100 kDa, which is in the range of other known fructosyltransferases and in line with the molecular weight of 86 kDa deduced of the amino acid sequence depicted in figure 1.

The invention further covers an inulosucrase according to the invention which, in the presence of sucrose, produces a inulin having β(2-1)-linked D-fructosyl units and fructo-oligosaccharides. Two different types of fructans, inulins and levans, exist in nature. Surprisingly, the novel inulosucrase expressed in *E. coli* Top10 cell synthesises a high molecular weight (>10⁷ Da) inulin and fructo-oligosaccharides, while in *Lactobacillus reuteri* culture supernatants, in addition to the fructo-oligosaccharides, a levan and not an inulin is found. This discrepancy can have several explanations: the inulosucrase gene may be silent in *Lactobacillus reuteri,* or may not be expressed in *Lactobacillus reuteri* under the conditions tested, or the inulosucrase may only synthesise fructo-oligosaccharides in its natural host, or the inulin polymer may be degraded shortly after synthesis, or may not be secreted and remains cell-associated, or the inulosucrase may have different activities in *Lactobacillus reuteri* and *E. coli* Top10 cells.

It was furthermore found that certain lactobacilli, in particular *Lactobacillus reuteri,* possess another fructosyltransferase, a levansucrase (FTFB), in addition to the inulosucrase described above. The N-terminal amino acid sequence of the fructosyltransferase purified from *Lactobacillus reuteri* supernatant was found to be QVESNNYNGVAEVNTERQANGQI (SEQ ID No. 6). Furthermore, three internal sequences were identified, namely (M)(A)HLDVWDSWPVQDP(V) (SEQ ID No. 7), NAGSIFGT(K) (SEQ ID No. 8), V(E)(E)VYSPKVSTLMASDEVE (SEQ ID No. 9). The N-terminal amino acid sequence could not be identified in the deduced inulosucrase sequence. Also the amino acid sequences of the three internal peptide fragments of the purified fructosyltransferase were not present in the putative inulosucrase sequence. Evidently, the inulosucrase gene does not encode the purified fructosyltransferase synthesizing the levan. The complete amino acid sequence of the levansucrase is shown in SEQ ID No. 11 and the nucleotide sequence is shown in SEQ ID No. 10. The levansucrase comprises a putative membrane anchor (see amino acids 761-765 in SEQ ID No. 11) and a putative membrane spanning domain (see amino acids 766-787 in SEQ ID No. 11). The fructan produced by the levansucrase was identified in the *Lactobacillus reuteri* culture supernatant as a linear (2→6)-β-D-fructofuranan with a molecular weight of 150 kDa. The purified enzyme also produces this fructan.

The invention also pertains to a process of producing an inulin-type of fructan as described above using isolated fructosyltransferases according to the invention and a suitable fructose source such as sucrose or raffinose. The fructosyltransferase enzyme may be isolated by conventional means from the culture of fructosyltransferase-positive lactobacilli, especially a *Lactobacillus reuteri,* or from a recombinant organism containing the fructosyltransferase gene or genes.

Additionally, the invention concerns a process of producing fructo-oligosaccharides containing the characteristic structure of the fructans described above using an isolated fructosyltransferase according to the invention. There is a growing interest in oligosaccharides derived from homopolysaccharides, for instance for prebiotic purposes. Several fructo- and gluco-oligosaccharides are known to stimulate the growth of bifido-bacteria in the human colon. Another way of producing fructo-oligosaccharides is by hydrolysis of the fructans described above. This hydrolysis can be performed by known hydrolysis methods such as enzymatic hydrolysis with enzymes such as levanase or inulinase or by acid hydrolysis. The fructo-oligosaccharides to be produced according to the invention preferably contain at least 2, more preferably at least 3, up to about 20 anhydrofructose units, optionally in addition to one or more other (glucose, galactose, etc.) units. These fructo-oligosaccharides are useful as prebiotics.

Chemically modified fructans and fructo-oligosaccharides can be obtained from the fructans described above. Chemical modification can be achieved by oxidation, phosphorylation, acylation, hydroxyalkylation, carboxymethylation, and amino-alkylation. Partial hydrolysis of fructans and modified fructans described above results in fructo-oligosaccharides, which can be used as bioactive carbohydrates or prebiotics.

The above mentioned modifications can be used either separately or in combination depending on the desired product. Furthermore, the degree of chemical modification is variable and depends on the intended use.

Use of a *Lactobacillus* strain capable of producing a levan, inulin or fructo-oligosaccharides or a mixture thereof, as a probiotic, is also covered by the invention. Preferably, the *Lactobacillus* strain is also capable of producing a glucan, especially a glucan as referred to above. The efficacy of some *Lactobacillus reuteri* strains as a probiotics has been demonstrated in various animals such as for instance poultry and humans. The administration of some *Lactobacillus reuteri* strains to pigs resulted in significantly lower serum total and LDL-cholesterol levels, while in children *Lactobacillus reuteri* is used as a therapeutic agent against acute diarrhea. For this and other reasons *Lactobacillus reuteri* strains, which were not reported to produce the glucans or fructans described herein, have been supplemented to commercially available probiotic products. The mode of action of *Lactobacillus reuteri* as a probiotic is still unclear. Preliminary studies indicated that gut colonization by *Lactobacillus reuteri* may be of importance. According to the invention, it was found that the mode of action of *Lactobacillus reuteri* as a probiotic may reside partly in the ability to produce polysaccharides. *Lactobacillus* strains, preferably *Lactobacillus reuteri* strains, and more preferably *Lactobacillus reuteri* strain LB 121 and other strains containing one or more fructosyltransferase genes encoding proteins capable of producing inulins, levans and/or fructo-oligosaccharides can thus advantageously be used as a probiotic. They can also, together with these polysaccharides, be used as a symbiotic.

### Examples

### Example 1: Isolation of DNA from Lactobacillus reuteri, nucleotide sequence analysis of the inulosucrase (ftfA) gene, construction of plasmids for expression of the inulosucrase gene in E. coli Top10, expression of the inulosucrase gene in E. coli Top10 and identification of the produced polysaccharides produced by the recombinant enzyme.

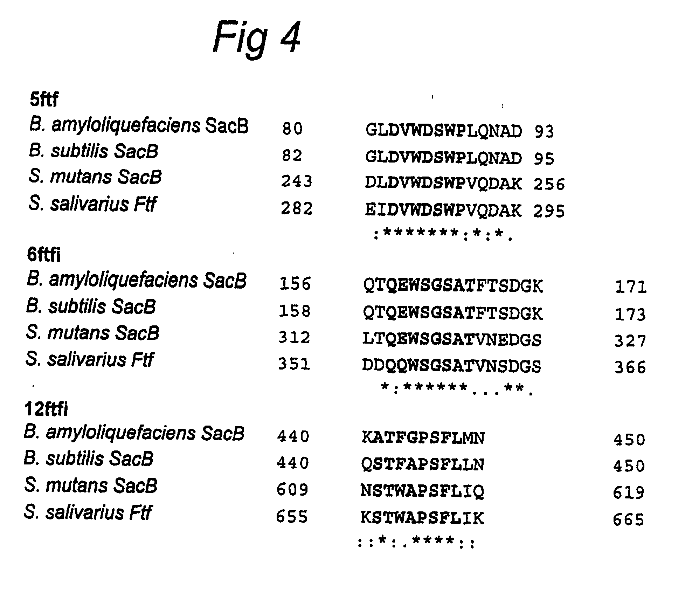

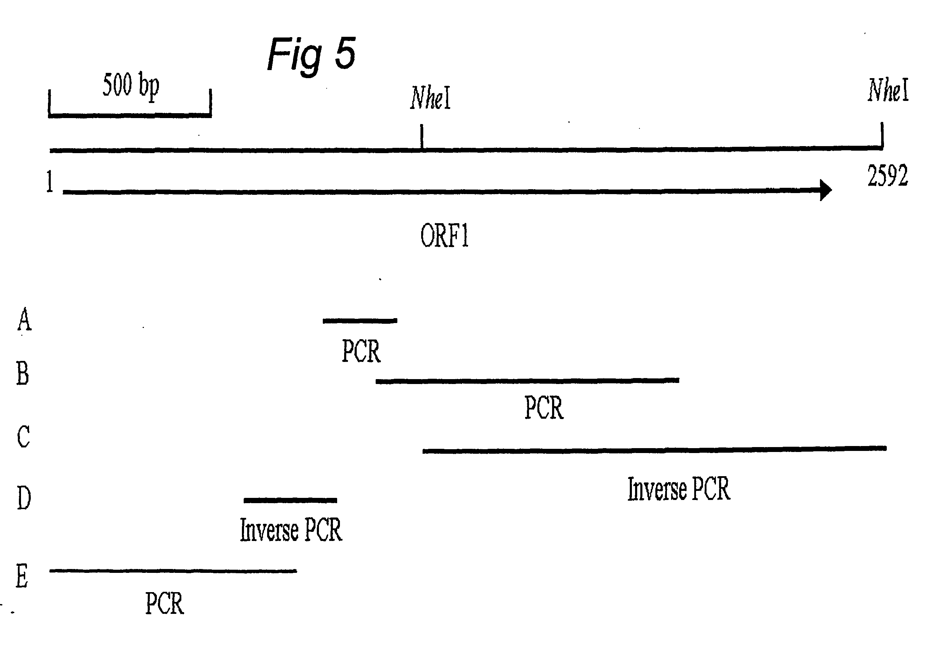

General procedures for cloning, DNA manipulations and agarose gel electrophoresis were essentially as described by Sambrook et al. (1989) Molecular cloning: a laboratory manual, 2nd ed. Cold Spring Harbour Laboratory Press, Cold Spring Harbour, New York. Restriction endonuclease digestions and ligations with T4 DNA ligase were performed as recommended by the suppliers. DNA was amplified by PCR techniques using ampliTAQ DNA polymerase (Perkin Elmer) or Pwo DNA polymerase. DNA fragments were isolated from agarose gels using the Qiagen extraction kit (Qiagen GMBH), following the instructions of the suppliers. *Lactobacillus reuteri* strain 121 (LMG 18388) was grown at 37°C in MRS medium (DIFCO) or in MRS-s medium (MRS medium containing 100 g/l sucrose instead of 20 g/l glucose). When fructo-oligosaccharides production was investigated phosphate was omitted and ammonium citrate was replaced by ammonium nitrate in the MRS-s medium. E. *coli* strains were grown aerobically at 37°C in LB medium, where appropriate supplemented with 50 µg/ml ampicillin (for selection of recombinant plasmids) or with 0.02% (w/v) arabinose (for induction of the inulosucrase gene).
Total DNA of *Lactobacillus reuteri* was isolated according to Verhasselt et al. (1989) FEMS Microbiol. Lett. 59, 135-140 as modified by Nagy et al. (1995) J. Bacteriol. 177, 676-687.
The inulosucrase gene was identified by amplification of chromosomal DNA of *Lactobacillus reuteri* with PCR using degenerated primers (5ftf, 6ftfi, and 12ftfi, see table 1) based on conserved amino acid sequences deduced from different bacterial fructosyltranferase genes (SacB of *Bacillus amyloliquefaciens,* SacB of *Bacillus subtilis, Streptococcus mutans* fructosyltransferase and *Streptococcus salivarius* fructosyltransferase, see figure 4) and *Lactobacillus reuteri* DNA as template. Using primers 5ftf and 6ftfi, an amplification product with the predicted size of about 234 bp was obtained (figure 5A). This 234 bp fragment was cloned in *E. coli* JM109 using the pCR2.1 vector and sequenced. Transformations were performed by electroporation using the BioRad gene pulser apparatus at 2.5 kV, 25 µF and 200 Ω, following the instructions of the manufacturer. Sequencing was performed according to the method of Sanger et al. (1977) Proc. Natl. Acad. Sci. USA 74, 5463-5467. Analysis of the obtained sequence data confirmed that part of a fructosyltransferase (*ftf*) gene had been isolated. The 234 bp amplified fragment was used to design primers 7ftf and 8ftfi (see table 1). PCR with the primers 7ftf and 12ftfi gave a product of the predicted size of 948 bp (see figure 5B); its sequence showed clear similarity with previously characterized fructosyltransferase genes. The 948 bp amplified fragment was used to design the primers ftfAC1(i) and ftfAC2(i) (see table 1) for inverse PCR. Using inverse PCR techniques a 1438 bp fragment of the inulosucrase gene was generated, including the 3' end of the inulosucrase gene (see figure 5C). The remaining 5' fragment of the inulosucrase gene was isloated with a combination of standard and inverse PCR techniques. Briefly, *Lactobacillus reuteri* DNA was cut with restriction enzyme *Xho*I and ligated. PCR with the primers 7ftf and 8ftfi, using the ligation product as a template, yielded a 290 bp PCR product which was cloned into pCR2.1 and sequenced. This revealed that primer 8ftfi had annealed aspecifically as well as specifically yielding the 290 bp product (see figure 5D).
At this time, the N-terminal amino acid sequence of a fructosyltransferase enzyme (FTFB) purified from the *Lactobacillus reuteri* strain 121 was obtained. This sequence consisted of the following 23 amino acids: QVESNNYNGVAEVNTERQANGQI (SEQ ID No. 6). The degenerated primer 19ftf (YNGVAEV) was designed on the basis of a part of this N-terminal peptide sequence and primer 20ftfi was designed on the 290 bp PCR product. PCR with primers l9ftf and 20ftfi gave a 754 bp PCR product (see figure 5E), which was cloned into pCR2.1 and sequenced. Both DNA strands of the entire fructosyltransferase gene were double sequenced. In this way the sequence of a 2.6 kb region of the *Lactobacillus reuteri* DNA, containing the inulosucrase gene and its surroundings were obtained.
The plasmids for expression of the inulosucrase gene in *E. coli* Top10 were constructed as described hereafter. A 2414 bp fragment, containing the inulosucrase gene starting at the first putative start codon at position 41, was generated by PCR, using primers ftfA1 and ftfA2i. Both primers contained suitable restriction enzyme recognition sites (a *Nco*I site at the 5'end of ftfA1 and a *Bgl*II site at the 3'end of ftfA2i). PCR with *Lactobacillus reuteri* DNA, Pwo DNA polymerase and primers ftfA1 and ftfA2i yielded the complete inulosucrase gene flanked by *Nco*I and *Bgl*II restriction sites. The PCR product with blunt ends was ligated directly into pCRbluntII-Topo. Using the *Nco*I and *Bgl*II restriction sites, the putative *ftfA* gene was cloned into the expression vector pBAD, downstream of the inducible arabinose promoter and in frame upstream of the Myc epitope and the His tag. The pBAD vector containing the inulosucrase gene (pSVH101) was transformed to *E. coli* Top10 and used to study inulosucrase expression. Correct construction of plasmid containing the complete inulosucrase gene was confirmed by restriction enzyme digestion analysis and by sequence analysis, showing an in frame cloning of the inulosucrase gene using the ribosomal binding site provided by the pBAD vector and the first putative start codon (at position 41) of inulosucrase (see figure 1).
Plasmid DNA of *E. coli* was isolated using the alkaline lysis method of Birnboim and Doly (1979) Nucleic Acids Res. 7, 1513-1523 or with a Qiagen plasmid kit following the instructions of the supplier. Cells of *E. coli* Top10 with pSVH101 were grown overnight in LB medium containing 0.02% (w/v) arabinose and were harvested by centrifugation. The pellet was washed with 25 mM sodium acetate buffer pH 5.4 and the suspension was centrifuged again. Pelleted cells were resuspended in 25 mM sodium acetate buffer pH 5.4. Cells were broken by sonication. Cell debris and intact cells were removed by centrifugation for 30 min at 4°C at 10,000xg and the resulting cell free extract was used in the enzyme assays.
The fructosyltranferase activities were determined at 37°C in reaction buffer (25 mM sodium acetate, pH 5.4, 1 mM CaCl₂, 100g/l sucrose) by monitoring the release of glucose from sucrose, by detecting fructo-oligosaccharides or by determining the amount of fructan polymer produced using *E. coli* cell free extracts or *Lactobacillus reuteri* culture supernatant as enzyme source. Sucrose, glucose and fructose were determined enzymatically using commercially available kits.
Fructan production by *Lactobacillus reuteri* was studied with cells grown in MRS-s medium. Product formation was also studied with cell-free extracts of *E. coli* containing the novel inulosucrase incubated in reaction buffer (1 mg protein/10 ml buffer, incubated overnight at 37°C). Fructans were collected by precipitation with ethanol. ¹H-NMR spectroscopy and methylation analysis were performed as described by van Geel-Schutten et al. (1999) Appl. Environ. Microbiol. 65, 3008-3014. The molecular weights of the fructans were determined by high performance size exclusion chromotography coupled on-line with a multi angle laser light scattering and a differential refractive index detector. Fructo-oligosaccharide synthesis was studied in *Lactobacillus reuteri* culture supernatants and in extracts of *E. coli* cells containing the novel inulosucrase incubated in reaction buffer (1 mg protein/10 ml buffer, incubated overnight at 37°C). Glucose and fructose were determined enzymatically as described above and fructo-oligosaccharides produced were analyzed using a Dionex column. The incubation mixtures were centrifuged for 30 min at 10,000xg and diluted 1:5 in a 100% DMSO solution prior to injection on a Dionex column. A digest of inulin (DP1-20) was used as a standard. Separation of compounds was achieved with anion-exchange chromatography on a CarboPac Pal column (Dionex) coupled to a CarboPac PA1 guard column (Dionex). Using a Dionex GP50 pump the following gradient was generated: % eluent B is 5% (0 min); 35% (10 min); 45% (20 min); 65% (50 min); 100% (54-60 min); 5% (61-65 min). Eluent A was 0.1 M NaOH and eluent B was 0.6 M NaAc in a 0.1 M NaOH solution. Compounds were detected using a Dionex ED40 electrochemical detector with an AU working electrode and a Ag/AgCl reference-electrode with a sensitivity of 300 nC. The pulse program used was: +0.1 Volt (0-0.4 s); +0.7 Volt (0.41-0.60 s); -0.1 Volt (0.61-1.00 s). Data were integrated using a Perkin Elmer Turbochrom data integration system. A different separation of compounds was done on a cation exchange column in the calcium form (Benson BCX4). As mobile phase Ca-EDTA in water (100 ppm) was used. The elution speed was 0.4 ml/min at a column temperature of 85°C. Detection of compounds was done by a refractive index (Jasco 830-RI) at 40°C. Quantification of compounds was achieved by using the software program Turbochrom (Perkin Elmer).
SDS-PAGE was performed according to Laemmli (1970) Nature 227, 680-685 using 7.5% polyacrylamide gels. After electrophoresis gels were stained with Coomassie Briljant Blue or an activity staining (Periodic Acid Schiff, PAS) was carried out as described by Van Geel-Schutten et al. (1999) Appl. Environ. Microbiol. 65, 3008-3014.

**Table 1 Nucleotide sequence of primers used in PCR reactions to identify the inulosucrase gene.**

| Primer name | Location (bp) | Nucleotide sequence (and SEQ ID No) |
|---|---|---|
| ftfAC1 | 1176 | CTG-ATA-ATA-ATG-GAA-ATG-TAT-CAC (SEQ ID No. 12) |
| ftfAC2i | 1243 | CAT-GAT-CAT-AAG-TTT-GGT-AGT-AAT-AG (SEQ ID No. 13) |
| ftfac1 | 1176 | GTG-ATA-CAT-TTC-CAT-TAT-TAT-CAG (SEQ ID No. 14) |
| ftfAC2 | 1243 | CTA-TTA-CTA-CCA-AAC-TTA-TGA-TCA-TG (SEQ ID No. 15) |
| ftfA1 | | |
| ftfA2i | | |
| 5ftf | 845 | GAY-GTN-TGG-GAY-WSN-TGG-GCC (SEQ ID No. 18) |
| 6ftfi | 1052 | GTN-GCN-SWN-CCN-SWC-CAY-TSY-TG (SEQ IID No. 19) |
| 7ftf | 1009 | GAA-TGT-AGG-TCC-AAT-TTT-TGG-C (SEQ ID No. 20) |
| 8ftfi | 864 | CCT-GTC-CGA-ACA-TCT-TGA-ACT-G (SEQ ID No. 21) |
| 12ftfi | 1934 | ARR-AAN-SWN-GGN-GCV-MAN-GTN-SW (SEQ ID No. 22) |
| 19ftf | 1 | TAY-AAY-GGN-GTN-GCN-GAR-GTN-AA (SEQ ID No. 23) |
| 20ftfi | 733 | CCG-ACC-ATC-TTG-TTT-GAT-TAA-C (SEQ ID No. 24) |

Listed from left to right are: primer name (i, inverse primer), location (in bp) in *ftfA* and the sequence from 5' to 3' according to IUB group codes (N=any base;M=A or C; R=A or G; W=A or T; S=C or G; Y=C or T; K=G or T; B=not A; D=not C; H=not G; and V=not T).

### Example 2: Adhesion of Lactobacillus reuteri strains to Caco-2 cell lines

The adhesion of *Lactobacillus reuteri* strains to Caco-2 cell lines was determined as described below. Firstly, a bacterial suspension was prepared as follows. *Lactobacillus reuteri* strains LB 121, 35-5 and *L. rhamnosus* LGG (a well known probiotic strain with good adhering properties) were cultured in MRS broth supplemented with 5 µl/ml of methyl-1,2-[³H]-thymidine at 37°C for 18-20 h before the adhesion assays. The cultures were harvested by centrifugation, washed with phosphate buffered saline (PBS) and resuspended in PBS or PBS supplemented with 30 g/l sucrose (see Table 3) to a final density of about 2 x 10⁹ cfu/ml. Prior to the adhesion assay, the cell suspensions in PBS with 30 g/l sucrose were incubated for 1 hour at 37°C, whereas the cell suspensions in PBS were kept on ice for 1 hour. After incubation at 37°C, the suspensions in PBS with sucrose were centrifuged and the cells were washed with and resuspended in PBS to a final density of about 2 x 10⁹ cfu/ml.
Caco-2 cells were cultured as follows. Subcultures of Caco-2 cells (ATCC, code HTB 37, human colon adenocarcinoma), stored as frozen stock cultures in liquid nitrogen were used for the adhesion tests. The Caco-2 cells were grown in culture medium consisting of Dulbecco's modified Eagle medium (DMEM), supplemented with heat-inactivated foetal calf serum (10% v/v), non-essential amino acids (1% v/v), L-glutamine (2mM) and gentamicin (50 µg/ml). About 2,000,000 cells were seeded in 75cm² tissue culture flasks containing culture medium and cultured in a humidified incubator at 37°C in air containing 5% CO₂. Near confluent Caco-2 cell cultures were harvested by trypsinisation and resuspended in culture medium. The number of cells was established using a Bürker-Türk counting chamber.

**Table 3: Incubation of the different Lactobacillus strains prior to the adhesion assays.**

| *Lactobacillus* strain | Extra incubation | Polysaccharide produced | Group |
|---|---|---|---|
| *reuteri* 121 | PBS sucrose, 37°C for 1 hr | glucan and fructan | As |
| *reuteri 35-5* | PBS sucrose, 37°C for 1 hr | glucan | Bs |
| *reuteri* K24 | PBS sucrose, 37°C for 1 hr | none | Cs |
| *reuteri* 121 | PBS on ice | none | D |
| *reuteri* DSM20016* | PBS on ice | none | E |
| *rhamnosus* GG | PBS on ice | none | F |

| | | | |
|---|---|---|---|
| * Type strain of *L. reuteri* | | | |

For the following experiments a Caco-2 monolayer transport system was used. Caco-2 cells cultured in a two-compartment transport system are commonly used to study the intestinal, epithelial permeability. In this system the Caco-2 cell differentiates into polarized columnar cells after reaching confluency. The Caco-2 system has been shown to simulate the passive and active transcellular tranport of electrolytes, sugars, amino acids and lipophilic compounds (Hillgren *et al.* 1995, Dulfer *et al.,* 1996, Duizer *et al.,* 1997). Also, a clear correlation between the *in vivo* absorption and the permeability across the monolayers of Caco-2 cells has been reported (Artursson and Karlsson, 1990). For the present transport studies, Caco-2 cells were seeded on semi-permeable filter inserts (12 wells Transwell plates, Costar) at ca. 100,000 cells per filter (growth area ± 1 cm² containing 2.5 ml culture medium). The cells on the insert were cultured for 17 to 24 days at 37°C in a humidified incubator containing 5% CO₂ in air. During this culture period the cells have been subjected to an enterocyte-like differentiation. Gentamycin was eliminated from the culture medium two days prior to the adhesion assays.
The adhesion assay was performed as follows. PBS was used as exposure medium. 25 µl of a bacterial suspension (2 x 10⁹ cfu/ml) were added to 0.5 ml medium. The apical side of the Caco-2 monolayers was incubated with the bacterial suspensions for 1 hour at 37°C. After incubation, remaining fluid was removed and the cells were washed three times with 1 ml PBS. Subsequently, the Caco-2 monolayers were digested overnight with 1 ml 0.1M NaOH, 1% SDS. The lysate was mixed with 10 ml Hionic Fluor scintillation liquid and the radioactivity was measured by liquid scintillation counting using a LKB/Wallac scintillation counter. As a control, the radioactivity of the bacterial suspensions was measured. For each test group, the percentage of bacteria attached to the monolayers was calculated. All adhesion tests were performed in quadruple. In Table 4 the results of the bacterial adhesion test to Caco-2 cellines are given. From the results can be concluded that the glucans and the fructans contribute to the adherence of *Lactobacillus reuteri* to Caco-2 cellines. This could indicate that *Lactobacillus reuteri* strains producing EPS possess improved probiotic characteristics or that *Lactobacillus reuteri* and its polysaccharides could function as an exellent symbiotic.

**Table 4: The results of the bacterial adhesion test to Caco-2 cellines.**

| | |
|---|---|
| Group (see Table 1) | % of bacteria bound to the monolayer |
| As | 6.5 |
| Bs | 5.7 |
| Cs | 1.8 |
| D | 2.3 |
| E | 0.9 |
| F | 1.3 |

### Description of the figures

Figure 1: SEQ ID No. 1; The deduced amino acid sequence of the novel inulosucrase of *Lactobacillus reuteri* (amino acid 1-789). Furthermore, the designations and orientation (< for 3' to 5' and > for 5' to 3') of the primers and the restriction enzymes used for (inverse) PCR, are shown at the right hand side. Putative start codons (ATG, at positions 41 and 68) and stop codon (TAA, at position 2435) are shown in bold. The positions of the primers used for PCR are shown in bold/underlined. The *Nhe*I restriction sites (at positions 1154 and 2592) used for inverse PCR are underlined. The primers used and their exact posotions in the inulosucrase sequence are shown in table 1. Starting at amino acid 690, the 20 PXX repeats are underlined. At amino acid 755 the LPXTG motif is underlined.
Figure 2: Dendrogram of bacterial and plant fructosyltransferases. The horizontal distances are a measure for the difference at the amino acid sequence level. 10% difference is indicated by the upper bar. Bootstrap values (in percentages) are given at the root of each tree. Fructosyltransferases of Gram positive bacteria are indicated in the lower half of the figure (*B. staerothermophilus* SurB; *B. amyloliquefaciens* SacB; *B. subtilis* SacB; *S. mutans* SacB; *L. reuteri* FtfA (inulosucrase); *S. salivarius* Ftf). Plant fructosyltransferases are indicated in the middle part of the figure (*Cynara scolymus* Ss-1ft; *Allium cepa* F-6gft; *Hordeum vulgare* Sf-6ft). Fructosyltransferases of Gram negative bacteria are shown in the upper part of the figure (*Z. mobilis* LevU; *Z. mobilis* SucE2; *Z. mobilis* SacB; *E. amylovora* Lcs; *A. diazotrophicus*LsdA).

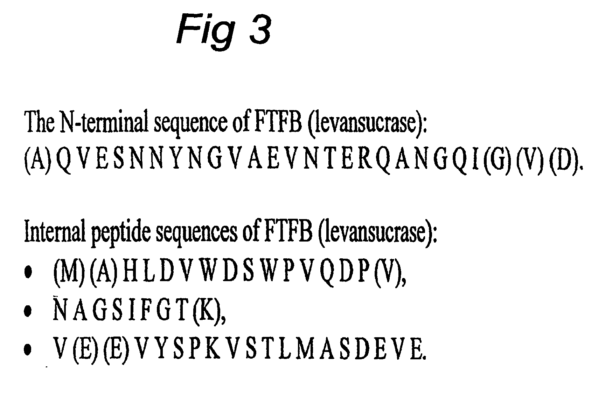

Figure 3: The N-terminal and three internal amino acid sequences of the novel levansucrase of *Lactobacillus reuteri.*
Figure 4: Parts of an alignment of the deduced amino acid sequences of some bacterial fructosyltransferase genes. Sequences in bold indicate the consensus sequences used to construct the degenerated primers 5ftf, 6ftfi and 12 ftfi. (*) indicates a position with a fully conserved amino acid residue. (:) indicates a position with a fully conserved 'strong' group: STA, NEQK, NHQK, NDEQ, QHRK, MILV, MILF, HY, FYW. (.) indicates a position with a fully conserved 'weaker' group: CSA, ATV, SAG, STNK, STPA, SGND, SNDEQK, NDEQHK, NEQHRK, FVLIM, HFY. Goups are according to the Pam250 residue weight matrix described by Altschul et al. (1990) J. Mol. Biol. 215, 403-410.
Figure 5: The strategy used for the isolation of the inulosucrase gene from *Lactobacillus reuteri* 121 chromosomal DNA.

## Claims

1. A protein having fructosyltransferase activity, the amino acid sequence of the protein exhibiting at least 75% amino acid identity, as determined by the BLAST algorithm, with the amino acid sequence of SEQ ID No. 1.

2. A protein according to claim 1, exhibiting at least 85% amino acid identity with the amino acid sequence of SEQ ID No. 1.

3. A protein according to claim 1 or 2, having at least 15 contiguous amino acids which are identical to 15 contiguous amino acids of the amino acid sequence of SEQ ID No. 1.

4. A protein according to any one of claims 1-3, which, in the presence of sucrose, produces an inulin having β(2-1) linked D-fructosyl units.

5. A protein according to any one of the preceding claims which is a recombinant protein.

6. A nucleotide sequence encoding a protein according to any one of the preceding claims.

7. A nucleic acid construct comprising the nucleotide sequence of claim 6, operationally linked to an expression-regulating nucleic acid sequence.

8. A recombinant host cell containing one or more copies of the nucleic acid construct according to claim 7.

9. A process of producing a fructosyltransferase, comprising culturing a host cell according to claim 8 in a culture medium, and recovering the protein from the culture medium or the cell-free extract.

10. A process of producing an oligosaccharide or polysaccharide containing fructosyl units, comprising subjecting a fructose source to the activity of a protein according to any one of claims 1-5, or a host cell according to claim 8.

11. Use of a *Lactobacillus* strain containing a protein according to any one of claims 1-5 and capable of producing an inulin, a levan or fructo-oligosaccharides, as a probiotic or synbiotic.

## Patentansprüche

1. Protein mit Fructosyltransferaseaktivität, wobei die Aminosäuresequenz des Proteins wenigstens 75 % Aminosäureidentität, bestimmt durch den BLAST-Algorithmus, mit der Aminosäuresequenz von SEQ ID Nr. 1 aufweist.

2. Protein nach Anspruch 1, das wenigstens 85 % Aminosäureidentität mit der Aminosäuresequenz von SEQ ID Nr. 1 aufweist.

3. Protein nach Anspruch 1 oder 2 mit wenigstens 15 aufeinander folgenden Aminosäuren, welche identisch zu 15 zusammenhängenden Aminosäuren der Aminosäuresequenz von SED ID Nr. 1 sind.

4. Protein nach einem der Ansprüche 1-3, welches in Gegenwart von Sucrose ein Inulin mit β(2-1)-verknüpften D-Fructosyleinheiten erzeugt.

5. Protein nach einem der vorangehenden Ansprüche, welches ein rekombinantes Protein ist.

6. Nucleotidsequenz, die ein Protein nach einem der vorangehenden Ansprüche kodiert.

7. Nucleinsäurekonstrukt umfassend die Nucleotidsequenz nach Anspruch 6, funktionell verbunden mit einer die Expression regulierenden Nucleinsäuresequenz.

8. Rekombinante Wirtszelle, enthaltend eine oder mehrere Kopien des Nucleinsäurekonstrukts nach Anspruch 7.

9. Verfahren zum Herstellen einer Fructosyltransferase, umfassend das Kultivieren einer Wirtszelle nach Anspruch 8 in einem Kulturmedium und das Gewinnen des Proteins aus dem Kulturmedium oder dem zellfreien Extrakt.

10. Verfahren zum Herstellen eines Oligosaccharids oder Polysaccharids, das Fructosyleinheiten enthält, umfassend das Aussetzen einer Fructosequelle der Aktivität eines Proteins nach einem der Ansprüche 1-5 oder einer Wirtszelle nach Anspruch 8.

11. Verwendung eines *Lactobacillus*-Stamms, der ein Protein nach einem der Ansprüche 1-5 enthält und im Stande ist, ein Inulin, ein Levan oder Fructo-Oligosaccharide zu erzeugen, als Probiotikum oder Synbiotikum.

## Revendications

1. Protéine ayant une activité fructosyl transférase, dans laquelle la séquence d'acides aminés de la protéine présente une identité d'au moins 75% avec la séquence d'acides aminés de SEQ ID n° 1, où l'identité en acides aminés est déterminée par l'algorithme de BLAST.

2. Protéine selon la revendication 1, présentant au moins 85 % d'identité, avec la séquence d'acides aminés SEQ ID n° 1.

3. Protéine selon la revendication 1 ou 2, ayant au moins 15 acides aminés contigus qui sont identiques à 15 acides aminés contigus de la séquence d'acides aminés SEQ ID n° 1.

4. Protéine selon l'une quelconque des revendications 1-3 qui, en présence de sucrose, produit une inuline ayant des unités D-fructosyl liées β(2-1).

5. Protéine selon l'une quelconque des revendications précédentes, qui est une protéine recombinée.

6. Séquence nucléotide codant une protéine selon l'une quelconque des revendications précédentes.

7. Construction d'acide nucléique comprenant la séquence nucléotide de la revendication 6, fonctionnellement liée à une séquence d'acides nucléiques régulant l'expression.

8. Cellule hôte recombinée contenant une ou plusieurs copies de la construction d'acide nucléique selon la revendication 7.

9. Procédé pour produire une fructosyltransférase, comprenant la mise en culture d'une cellule hôte selon la revendication 8 dans un milieu de culture, et la récupération de la protéine à partir du milieu de culture ou l'extrait exempt de cellule.

10. Procédé pour produire un oligosaccharide ou polysaccharide contenant des unités fructosyle, comprenant l'exposition d'une source de fructose à l'activité d'une protéine selon l'une quelconque des revendications 1-5, ou une cellule hôte selon la revendication 8.

11. Utilisation d'une souche *Lactobacillus* contenant une protéine selon l'une quelconque des revendications 1-5 et qui est capable de produire une inuline, un levan ou des fructo-oligosaccharides, en tant que probiotique ou synbiotique.


### SEQUENCE LISTING

<110> TNO
<120> Novel fructosyltransferases
<130> Novel fructosyltransferases
<140>
   <141>
<150> 00201872.9
   <151> 2000-05-25
<150> 01200049.3
   <151> 2001-01-09
<160> 26
<170> PatentIn Ver. 2.1
<210> 1
   <211> 789
   <212> PRT
   <213> Lactobacillus reuteri
<220>
   <221> ACT_SITE
   <222> (263)
   <223> Putative catalytic amino acid residue
<220>
   <221> ACT_SITE
   <222> (330)
   <223> Putative catalytic amino acid residue
<220>
   <221> ACT_SITE
   <222> (415)
   <223> Putative catalytic amino acid residue
<220>
   <221> ACT_SITE
   <222> (431)
   <223> Putative catalytic amino acid residue
<220>
   <221> ACT_SITE
   <222> (511)
   <223> Putative catalytic amino acid residue
<220>
   <221> ACT_SITE
   <222> (514)
   <223> Putative catalytic amino acid residue
<220>
   <221> ACT_SITE
   <222> (532)
   <223> Putative catalytic amino acid residue
<220>
   <221> ACT_SITE
   <222> (551)
   <223> Putative catalytic amino acid residue
<220>
   <221> SIGNAL
   <222> (1)..(21)
   <223> Putative signal sequence
<220>
   <221> DOMAIN
   <222> (755)..(759)
   <223> Putative cell wall anchor amino acid signal
<220>
   <221> REPEAT
   <222> (690) .. (749)
   <223> PXX repeat (20 -fold)
<400> 1
   Met Tyr Lys Ser Gly Lys Asn Trp Ala Val Val Thr Leu Ser Thr Ala
   1               5                   10                  15
   Ala Leu Val Phe Gly Ala Thr Thr Val Asn Ala Ser Ala Asp Thr Asn
               20                  25                  30
   Ile Glu Asn Asn Asp Ser Ser Thr Val Gln Val Thr Thr Gly Asp Asn
           35                  40                  45
   Asp Ile Ala Val Lys Ser Val Thr Leu Gly Ser Gly Gln Val Ser Ala
       50                  55                  60
   Ala Ser Asp Thr Thr Ile Arg Thr Ser Ala Asn Ala Asn Ser Ala Ser
   65                  70                  75                  80
   Ser Ala Ala Asn Thr Gln Asn Ser Asn Ser Gln Val Ala Ser Ser Ala
                   85                  90                  95
   Ala Ile Thr Ser Ser Thr Ser Ser Ala Ala Ser Leu Asn Asn Thr Asp
               100                 105                 110
   Ser Lys Ala Ala Gln Glu Asn Thr Asn Thr Ala Lys Asn Asp Asp Thr
           115                 120                 125
   Gln Lys Ala Ala Pro Ala Asn Glu Ser Ser Glu Ala Lys Asn Glu Pro
       130                 135                 140
   Ala Val Asn Val Asn Asp Ser Ser Ala Ala Lys Asn Asp Asp Gln Gln
   145                 150                 155                 160
   Ser Ser Lys Lys Asn Thr Thr Ala Lys Leu Asn Lys Asp Ala Glu Asn
                   165                 170                 175
   Val Val Lys Lys Ala Gly Ile Asp Pro Asn Ser Leu Thr Asp Asp Gln
               180                 185                 190
   Ile Lys Ala Leu Asn Lys Met Asn Phe Ser Lys Ala Ala Lys Ser Gly
           195                 200                 205
   Thr Gln Met Thr Tyr Asn Asp Phe Gln Lys Ile Ala Asp Thr Leu Ile
       210                 215                 220
   Lys Gln Asp Gly Arg Tyr Thr Val Pro Phe Phe Lys Ala Ser Glu Ile
   225                 230                 235                 240
   Lys Asn Met Pro Ala Ala Thr Thr Lys Asp Ala Gln Thr Asn Thr Ile
                   245                 250                 255
   Glu Pro Leu Asp Val Trp Asp Ser Trp Pro Val Gln Asp Val Arg Thr
               260                 265                 270
   Gly Gln Val Ala Asn Trp Asn Gly Tyr Gln Leu Val Ile Ala Met Met
           275                 280                 285
   Gly Ile Pro Asn Gln Asn Asp Asn His Ile Tyr Leu Leu Tyr Asn Lys
       290                 295                 300
   Tyr Gly Asp Asn Glu Leu Ser His Trp Lys Asn Val Gly Pro Ile Phe
   305                 310                 315                 320
   Gly Tyr Asn Ser Thr Ala Val Ser Gln Glu Trp Ser Gly Ser Ala Val
                   325                 330                 335
   Leu Asn Ser Asp Asn Ser Ile Gln Leu Phe Tyr Thr Arg Val Asp Thr
   340 345 350
   Ser Asp Asn Asn Thr Asn His Gln Lys Ile Ala Ser Ala Thr Leu Tyr
   355 360 365
   Leu Thr Asp Asn Asn Gly Asn Val Ser Leu Ala Gln Val Arg Asn Asp
   370 375 380
   Tyr Ile Val Phe Glu Gly Asp Gly Tyr Tyr Tyr Gln Thr Tyr Asp Gln
   385 390 395 400
   Trp Lys Ala Thr Asn Lys Gly Ala Asp Asn Ile Ala Met Arg Asp Ala
   405 410 415
   His Val Ile Glu Asp Gly Asn Gly Asp Arg Tyr Leu Val Phe Glu Ala
   420 425 430
   Ser Thr Gly Leu Glu Asn Tyr Gln Gly Glu Asp Gln Ile Tyr Asn Trp
   435 440 445
   Leu Asn Tyr Gly Gly Asp Asp Ala Phe Asn Ile Lys Ser Leu Phe Arg
   450 455 460
   Ile Leu Ser Asn Asp Asp Ile Lys Ser Arg Ala Thr Trp Ala Asn Ala
   465 470 475 480
   Ala Ile Gly Ile Leu Lys Leu Asn Lys Asp Glu Lys Asn Pro Lys Val
   485 490 495
   Ala Glu Leu Tyr Ser Pro Leu Ile Ser Ala Pro Met Val Ser Asp Glu
   500 505 510
   Ile Glu Arg Pro Asn Val Val Lys Leu Gly Asn Lys Tyr Tyr Leu Phe
   515 520 525
   Ala Ala Thr Arg Leu Asn Arg Gly Ser Asn Asp Asp Ala Trp Met Asn
   530 535 540
   Ala Asn Tyr Ala Val Gly Asp Asn Val Ala Met Val Gly Tyr Val Ala
   545 550 555 560
   Asp Ser Leu Thr Gly Ser Tyr Lys Pro Leu Asn Asp Ser Gly Val Val
   565 570 575
   Leu Thr Ala Ser Val Pro Ala Asn Trp Arg Thr Ala Thr Tyr Ser Tyr
   580 585 590
   Tyr Ala Val Pro Val Ala Gly Lys Asp Asp Gln Val Leu Val Thr Ser
           595                 600                 605
   Tyr Met Thr Asn Arg Asn Gly Val Ala Gly Lys Gly Met Asp Ser Thr
       610                 615                 620
   Trp Ala Pro Ser Phe Leu Leu Gln Ile Asn Pro Asp Asn Thr Thr Thr
   625                 630                 635                 640
   Val Leu Ala Lys Met Thr Asn Gln Gly Asp Trp Ile Trp Asp Asp Ser
                   645                 650                 655
   Ser Glu Asn Leu Asp Met Ile Gly Asp Leu Asp Ser Ala Ala Leu Pro
               660                 665                 670
   Gly Glu Arg Asp Lys Pro Val Asp Trp Asp Leu Ile Gly Tyr Gly Leu
           675                 680                 685
   Lys Pro His Asp Pro Ala Thr Pro Asn Asp Pro Glu Thr Pro Thr Thr
       690                 695                 700
   Pro Glu Thr Pro Glu Thr Pro Asn Thr Pro Lys Thr Pro Lys Thr Pro
   705                 710                 715                 720
   Glu Asn Pro Gly Thr Pro Gln Thr Pro Asn Thr Pro Asn Thr Pro Glu
                   725                 730                 735
   Ile Pro Leu Thr Pro Glu Thr Pro Lys Gln Pro Glu Thr Gln Thr Asn
               740                 745                 750
   Asn Arg Leu Pro Gln Thr Gly Asn Asn Ala Asn Lys Ala Met Ile Gly
           755                 760                 765
   Leu Gly Met Gly Thr Leu Leu Ser Met Phe Gly Leu Ala Glu Ile Asn
       770                 775                 780
   Lys Arg Arg Phe Asn
   785
<210> 2
   <211> 2367
   <212> DNA
   <213> Lactobacillus reuteri
<400> 2
   atgtataaaa gcggtaaaaa ttgggcagtc gttacactct cgactgctgc gctggtattt 60
   ggtgcaacaa ctgtaaatgc atccgcggac acaaatattg aaaacaatga ttcttctact 120
   gtacaagtta caacaggtga taatgatatt gctgttaaaa gtgtgacact tggtagtggt 180
   caagttagtg cagctagtga tacgactatt agaacttctg ctaatgcaaa tagtgcttct 240
   tctgccgcta atacacaaaa ttctaacagt caagtagcaa gttctgctgc aataacatca 300
   tctacaagtt ccgcagcttc attaaataac acagatagta aagcggctca agaaaatact 360
   aatacagcca aaaatgatga cacgcaaaaa gctgcaccag ctaacgaatc ttctgaagct 420
   aaaaatgaac cagctgtaaa cgttaatgat tcttcagctg caaaaaatga tgatcaacaa 480
   tccagtaaaa agaatactac cgctaagtta aacaaggatg ctgaaaacgt tgtaaaaaag 540
   gcgggaattg atcctaacag tttaactgat gaccagatta aagcattaaa taagatgaac 600
   ttctcgaaag ctgcaaagtc tggtacacaa atgacttata atgatttcca aaagattgct 660
   gatacgttaa tcaaacaaga tggtcggtac acagttccat tctttaaagc aagtgaaatc 720
   aaaaatatgc ctgccgctac aactaaagat gcacaaacta atactattga acctttagat 780
   gtatgggatt catggccagt tcaagatgtt cggacaggac aagttgctaa ttggaatggc 840
   tatcaacttg tcatcgcaat gatgggaatt ccaaaccaaa atgataatca tatctatctc 900
   ttatataata agtatggtga taatgaatta agtcattgga agaatgtagg tccaattttt 960
   ggctataatt ctaccgcggt ttcacaagaa tggtcaggat cagctgtttt gaacagtgat 1020
   aactctatcc aattatttta tacaagggta gacacgtctg ataacaatac caatcatcaa 1080
   aaaattgcta gcgctactct ttatttaact gataataatg gaaatgtatc actcgctcag 1140
   gtacgaaatg actatattgt atttgaaggt gatggctatt actaccaaac ttatgatcaa 1200
   tggaaagcta ctaacaaagg tgccgataat attgcaatgc gtgatgctca tgtaattgaa 1260
   gatggtaatg gtgatcggta ccttgttttt gaagcaagta ctggtttgga aaattatcaa 1320
   ggcgaggacc aaatttataa ctggttaaat tatggcggag atgacgcatt taatatcaag 1380
   agcttattta gaattctttc caatgatgat attaagagtc gggcaacttg ggctaatgca 1440
   gctatcggta tcctcaaact aaataaggac gaaaagaatc ctaaggtggc agagttatac 1500
   tcaccattaa tttctgcacc aatggtaagc gatgaaattg agcgaccaaa tgtagttaaa 1560
   ttaggtaata aatattactt atttgccgct acccgtttaa atcgaggaag taatgatgat 1620
   gcttggatga atgctaatta tgccgttggt gataatgttg caatggtcgg atatgttgct 1680
   gatagtctaa ctggatctta taagccatta aatgattctg gagtagtctt gactgcttct 1740
   gttcctgcaa actggcggac agcaacttat tcatattatg ctgtccccgt tgccggaaaa 1800
   gatgaccaag tattagttac ttcatatatg actaatagaa atggagtagc gggtaaagga 1860
   atggattcaa cttgggcacc gagtttctta ctacaaatta acccggataa cacaactact 1920
   gttttagcta aaatgactaa tcaaggggat tggatttggg atgattcaag cgaaaatctt 1980
   gatatgattg gtgatttaga ctccgctgct ttacctggcg aacgtgataa acctgttgat 2040
   tgggacttaa ttggttatgg attaaaaccg catgatcctg ctacaccaaa tgatcctgaa 2100
   acgccaacta caccagaaac ccctgagaca cctaatactc ccaaaacacc aaagactcct 2160
   gaaaatcctg ggacacctca aactcctaat acacctaata ctccggaaat tcctttaact 2220
   ccagaaacgc ctaagcaacc tgaaacccaa actaataatc gtttgccaca aactggaaat 2280
   aatgccaata aagccatgat tggcctaggt atgggaacat tgcttagtat gtttggtctt 2340
   gcagaaatta acaaacgtcg atttaac                                     2367
<210> 3
   <211> 2394
   <212> DNA
   <213> Lactobacillus reuteri
<400> 3
   atgctagaac gcaaggaaca taaaaaaatg tataaaagcg gtaaaaattg ggcagtcgtt 60
   acactctcga ctgctgcgct ggtatttggt gcaacaactg taaatgcatc cgcggacaca 120
   aatattgaaa acaatgattc ttctactgta caagttacaa caggtgataa tgatattgct 180
   gttaaaagtg tgacacttgg tagtggtcaa gttagtgcag ctagtgatac gactattaga 240
   acttctgcta atgcaaatag tgcttcttct gccgctaata cacaaaattc taacagtcaa 300
   gtagcaagtt ctgctgcaat aacatcatct acaagttccg cagcttcatt aaataacaca 360
   gatagtaaag cggctcaaga aaatactaat acagccaaaa atgatgacac gcaaaaagct 420
   gcaccagcta acgaatcttc tgaagctaaa aatgaaccag ctgtaaacgt taatgattct 480
   tcagctgcaa aaaatgatga tcaacaatcc agtaaaaaga atactaccgc taagttaaac 540
   aaggatgctg aaaacgttgt aaaaaaggcg ggaattgatc ctaacagttt aactgatgac 600
   cagattaaag cattaaataa gatgaacttc tcgaaagctg caaagtctgg tacacaaatg 660
   acttataatg atttccaaaa gattgctgat acgttaatca aacaagatgg tcggtacaca 720
   gttccattct ttaaagcaag tgaaatcaaa aatatgcctg ccgctacaac taaagatgca 780
   caaactaata ctattgaacc tttagatgta tgggattcat ggccagttca agatgttcgg 840
   acaggacaag ttgctaattg gaatggctat caacttgtca tcgcaatgat gggaattcca 900
   aaccaaaatg ataatcatat ctatctctta tataataagt atggtgataa tgaattaagt 960
   cattggaaga atgtaggtcc aatttttggc tataattcta ccgcggtttc acaagaatgg 1020
   tcaggatcag ctgttttgaa cagtgataac tctatccaat tattttatac aagggtagac 1080
   acgtctgata acaataccaa tcatcaaaaa attgctagcg ctactcttta tttaactgat 1140
   aataatggaa atgtatcact cgctcaggta cgaaatgact atattgtatt tgaaggtgat 1200
   ggctattact accaaactta tgatcaatgg aaagctacta acaaaggtgc cgataatatt 1260
   gcaatgcgtg atgctcatgt aattgaagat ggtaatggtg atcggtacct tgtttttgaa 1320
   gcaagtactg gtttggaaaa ttatcaaggc gaggaccaaa tttataactg gttaaattat 1380
   ggcggagatg acgcatttaa tatcaagagc ttatttagaa ttctttccaa tgatgatatt 1440
   aagagtcggg caacttgggc taatgcagct atcggtatcc tcaaactaaa taaggacgaa 1500
   aagaatccta aggtggcaga gttatactca ccattaattt ctgcaccaat ggtaagcgat 1560
   gaaattgagc gaccaaatgt agttaaatta ggtaataaat attacttatt tgccgctacc 1620
   cgtttaaatc gaggaagtaa tgatgatgct tggatgaatg ctaattatgc cgttggtgat 1680
   aatgttgcaa tggtcggata tgttgctgat agtctaactg gatcttataa gccattaaat 1740
   gattctggag tagtcttgac tgcttctgtt cctgcaaact ggcggacagc aacttattca 1800
   tattatgctg tccccgttgc cggaaaagat gaccaagtat tagttacttc atatatgact 1860
   aatagaaatg gagtagcggg taaaggaatg gattcaactt gggcaccgag tttcttacta 1920
   caaattaacc cggataacac aactactgtt ttagctaaaa tgactaatca aggggattgg 1980
   atttgggatg attcaagcga aaatcttgat atgattggtg atttagactc cgctgcttta 2040
   cctggcgaac gtgataaacc tgttgattgg gacttaattg gttatggatt aaaaccgcat 2100
   gatcctgcta caccaaatga tcctgaaacg ccaactacac cagaaacccc tgagacacct 2160
   aatactccca aaacaccaaa gactcctgaa aatcctggga cacctcaaac tcctaataca 2220
   cctaatactc cggaaattcc tttaactcca gaaacgccta agcaacctga aacccaaact 2280
   aataatcgtt tgccacaaac tggaaataat gccaataaag ccatgattgg cctaggtatg 2340
   ggaacattgc ttagtatgtt tggtcttgca gaaattaaca aacgtcgatt taac 2394
<210> 4
   <211> 2592
   <212> DNA
   <213> Lactobacillus reuteri
<220>
   <221> RBS
   <222> (29)..(32)
<220>
   <221> RBS
   <222> (54)..(57)
<220>
   <221> misc_signal
   <222> (1)..(67)
   <223> Putative expression-regulating region
<220>
   <221> misc_signal
   <222> (2438)..(2592)
   <223> Putative expression-regulating region
<400> 4
   tacaatgggg tggcggaggt gaagaaacgg ggttacttct atgctagaac gcaaggaaca 60
   taaaaaaatg tataaaagcg gtaaaaattg ggcagtcgtt acactctcga ctgctgcgct 120
   ggtatttggt gcaacaactg taaatgcatc cgcggacaca aatattgaaa acaatgattc 180
   ttctactgta caagttacaa caggtgataa tgatattgct gttaaaagtg tgacacttgg 240
   tagtggtcaa gttagtgcag ctagtgatac gactattaga acttctgcta atgcaaatag 300
   tgcttcttct gccgctaata cacaaaattc taacagtcaa gtagcaagtt ctgctgcaat 360
   aacatcatct acaagttccg cagcttcatt aaataacaca gatagtaaag cggctcaaga 420
   aaatactaat acagccaaaa atgatgacac gcaaaaagct gcaccagcta acgaatcttc 480
   tgaagctaaa aatgaaccag ctgtaaacgt taatgattct tcagctgcaa aaaatgatga 540
   tcaacaatcc agtaaaaaga atactaccgc taagttaaac aaggatgctg aaaacgttgt 600
   aaaaaaggcg ggaattgatc ctaacagttt aactgatgac cagattaaag cattaaataa 660
   gatgaacttc tcgaaagctg caaagtctgg tacacaaatg acttataatg atttccaaaa 720
   gattgctgat acgttaatca aacaagatgg tcggtacaca gttccattct ttaaagcaag 780
   tgaaatcaaa aatatgcctg ccgctacaac taaagatgca caaactaata ctattgaacc 840
   tttagatgta tgggattcat ggccagttca agatgttcgg acaggacaag ttgctaattg 900
   gaatggctat caacttgtca tcgcaatgat gggaattcca aaccaaaatg ataatcatat 960
   ctatctctta tataataagt atggtgataa tgaattaagt cattggaaga atgtaggtcc 1020
   aatttttggc tataattcta ccgcggtttc acaagaatgg tcaggatcag ctgttttgaa 1080
   cagtgataac tctatccaat tattttatac aagggtagac acgtctgata acaataccaa 1140
   tcatcaaaaa attgctagcg ctactcttta tttaactgat aataatggaa atgtatcact 1200
   cgctcaggta cgaaatgact atattgtatt tgaaggtgat ggctattact accaaactta 1260
   tgatcaatgg aaagctacta acaaaggtgc cgataatatt gcaatgcgtg atgctcatgt 1320
   aattgaagat ggtaatggtg atcggtacct tgtttttgaa gcaagtactg gtttggaaaa 1380
   ttatcaaggc gaggaccaaa tttataactg gttaaattat ggcggagatg acgcatttaa 1440
   tatcaagagc ttatttagaa ttctttccaa tgatgatatt aagagtcggg caacttgggc 1500
   taatgcagct atcggtatcc tcaaactaaa taaggacgaa aagaatccta aggtggcaga 1560
   gttatactca ccattaattt ctgcaccaat ggtaagcgat gaaattgagc gaccaaatgt 1620
   agttaaatta ggtaataaat attacttatt tgccgctacc cgtttaaatc gaggaagtaa 1680
   tgatgatgct tggatgaatg ctaattatgc cgttggtgat aatgttgcaa tggtcggata 1740
   tgttgctgat agtctaactg gatcttataa gccattaaat gattctggag tagtcttgac 1800
   tgcttctgtt cctgcaaact ggcggacagc aacttattca tattatgctg tccccgttgc 1860
   cggaaaagat gaccaagtat tagttacttc atatatgact aatagaaatg gagtagcggg 1920
   taaaggaatg gattcaactt gggcaccgag tttcttacta caaattaacc cggataacac 1980
   aactactgtt ttagctaaaa tgactaatca aggggattgg atttgggatg attcaagcga 2040
   aaatcttgat atgattggtg atttagactc cgctgcttta cctggcgaac gtgataaacc 2100
   tgttgattgg gacttaattg gttatggatt aaaaccgcat gatcctgcta caccaaatga 2160
   tcctgaaacg ccaactacac cagaaacccc tgagacacct aatactccca aaacaccaaa 2220
   gactcctgaa aatcctggga cacctcaaac tcctaataca cctaatactc cggaaattcc 2280
   tttaactcca gaaacgccta agcaacctga aacccaaact aataatcgtt tgccacaaac 2340
   tggaaataat gccaataaag ccatgattgg cctaggtatg ggaacattgc ttagtatgtt 2400
   tggtcttgca gaaattaaca aacgtcgatt taactaaata ctttaaaata aaaccgctaa 2460
   gccttaaatt cagcttaacg gttttttatt ttaaaagttt ttattgtaaa aaagcgaatt 2520
   atcattaata ctaatgcaat tgttgtaaga ccttacgaca gtagtaacaa tgaatttgcc 2580
   catctttgtc gg                                                     2592
<210> 5
   <211> 5
   <212> PRT
   <213> Lactobacillus reuteri
<400> 5
   Leu Pro Xaa Thr Gly
   1               5
<210> 6
   <211> 23
   <212> PRT
   <213> Lactobacillus reuteri
<400> 6
   Gln Val Glu Ser Asn Asn Tyr Asn Gly Val Ala Glu Val Asn Thr Glu
   1               5                   10                  15
   Arg Gln Ala Asn Gly Gln Ile
               20
<210> 7
   <211> 16
   <212> PRT
   <213> Lactobacillus reuteri
<400> 7
   Met Ala His Leu Asp Val Trp Asp Ser Trp Pro Val Gln Asp Pro Val
   1 5 10 15
<210> 8
   <211> 9
   <212> PRT
   <213> Lactobacillus reuteri
<400> 8
   Asn Ala Gly Ser Ile Phe Gly Thr Lys
   1 5
<210> 9
   <211> 19
   <212> PRT
   <213> Lactobacillus reuteri
<400> 9
   Val Glu Glu Val Tyr Ser Pro Lys Val Ser Thr Leu Met Ala Ser Asp
   1 5 10 15
   Glu Val Glu
<210> 10
   <211> 4634
   <212> DNA
   <213> Lactobacillus reuteri
<220>
   <221> CDS
   <222> (1220)..(3598)
<220>
   <221> RBS
   <222> (1205)..(1210)
<400> 10
   gttaacaaag acaaaatttt atataattct tcaaattaaa tttcccactg taagaacata 60
   aatgggtacc tgtttgatgg gaataatata tttgtaacta accggccggc acctctttct 120
   aatgtgccta ggatgcataa tggatgtaaa ttactagatg gcggttttta tacattaacc 180
   tcgcaggaga gaaaagaagc aattagtaag gatccatatg cagataaatt tattaggcct 240
   tatttaggtg ctaaaaattt cattcatgga actgctaggt actgtatttg gttaaaggac 300
   gcaaacccga aagatatcca tcaatcgcca tttatactgg atagaatcaa taaagtagcg 360
   gaattcagat cgcagcaaaa aagtaaagat acacaaaaat atgcaaaacg gcccatgcta 420
   acaacacgac ttgcctatta tagccacgat gtacatacgg atatgctgat agtacctgca 480
   acatcatcgc aacgtagaga atatcttcca attggatatg tttcagaaaa gaatattgtg 540
   tcttattcac taatgctaat ccccaatgct agtaatttta atttcggtat tctagaatct 600
   aaagttcact atatttggtt aaaaaacttt tgcggtcggt tgaagtccga ttatcgttat 660
   tcaaacacta ttatttataa taatttccct tggccgactg ttggtgacaa gccaggamca 720
   acaccatctc tgacactcgc tcaaggtata ttaaatactc gcaagctcta tccagacagc 780
   tcactggctg atctttatga tccactaaca atgccragtt gaactcgtaa agctcatgaa 840
   gccaatgata aagctgttct taaagcatat ggattgagcc ctaaagctac tgagcaagaa 900
   atcgtagaac atctatttaa gatgtatgaa aaactgacta aaggtgaaag ataactttgt 960
   aaaaccaata ttttataaag acagtaaatg ttaatttgat aaaaacatat atttaataaa 1020
   caaaagtgat ataatcaagt agttctttgt attacaaaat acatttaata tctctcagca 1080
   ttttgcatac tgggagattt tttattgaca aattgtttga aagtgcttat gatgaaaccg 1140
   tgtagaaact aattcaattt gataaacgtt agacatttct gaggaggaag tcattttgga 1200
   gtacaaagaa cataagaaa atg tat aaa gtc ggc aag aat tgg gcc gtt gct  1252
                        Met Tyr Lys Val Gly Lys Asn Trp Ala Val Ala
                         1               5                  10
   aca ttg gta tca gct tca att tta atg gga ggg gtt gta acc gct cat   1300
   Thr Leu Val Ser Ala Ser Ile Leu Met Gly Gly Val Val Thr Ala His
               15                  20                  25
   gct gat caa gta gaa agt aac aat tac aac ggt gtt gct gaa gtt aat   1348
   Ala Asp Gln Val Glu Ser Asn Asn Tyr Asn Gly Val Ala Glu Val Asn
           30                  35                  40
   act gaa cgt caa gct aat ggt caa att ggc gta gat gga aaa att att   1396
   Thr Glu Arg Gln Ala Asn Gly Gln Ile Gly Val Asp Gly Lys Ile Ile
        45                  50                  55
   agt gct aac agt aat aca acc agt ggc tcg aca aat caa gaa tca tct    1444
   Ser Ala Asn Ser Asn Thr Thr Ser Gly Ser Thr Asn Gln Glu Ser Ser
    60                  65                  70                  75
   gct act aac aat act gaa aat gct gtt gtt aat gaa agc aaa aat act    1492
   Ala Thr Asn Asn Thr Glu Asn Ala Val Val Asn Glu Ser Lys Asn Thr
                    80                  85                  90
   aac aat act gaa aat gct gtt gtt aat gaa aac aaa aat act aac aat    1540
   Asn Asn Thr Glu Asn Ala Val Val Asn Glu Asn Lys Asn Thr Asn Asn
                95                 100                 105
   act gaa aat gct gtt gtt aat gaa aac aaa aat act aac aac aca gaa    1588
   Thr Glu Asn Ala Val Val Asn Glu Asn Lys Asn Thr Asn Asn Thr Glu
           110                 115                 120
   aac gat aat agt caa tta aag tta act aat aat gaa caa cca tca gcc    1636
   Asn Asp Asn Ser Gln Leu Lys Leu Thr Asn Asn Glu Gln Pro Ser Ala
       125                 130                 135
   gct act caa gca aac ttg aag aag cta aat cct caa gct gct aag gct    1684
   Ala Thr Gln Ala Asn Leu Lys Lys Leu Asn Pro Gln Ala Ala Lys Ala
   140                 145                 150                 155
   gtt caa aat gcc aag att gat gcc ggt agt tta aca gat gat caa att    1732
   Val Gln Asn Ala Lys Ile Asp Ala Gly Ser Leu Thr Asp Asp Gln Ile
                   160                 165                 170
   aat gaa tta aat aag att aac ttc tct aag tct gct gaa aag ggt gca    1780
   Asn Glu Leu Asn Lys Ile Asn Phe Ser Lys Ser Ala Glu Lys Gly Ala
               175                 180                 185
   aaa ttg acc ttt aag gac tta gag ggg att ggt aat gct att gtt aag    1828
   Lys Leu Thr Phe Lys Asp Leu Glu Gly Ile Gly Asn Ala Ile Val Lys
           190                 195                 200
   caa gat cca caa tat gct att cct tat tct aat gct aag gaa atc aag    1876
   Gln Asp Pro Gln Tyr Ala Ile Pro Tyr Ser Asn Ala Lys Glu Ile Lys
       205                 210                 215
   aat atg cct gca aca tac act gta gat gcc caa aca ggt aag atg gct    1924
   Asn Met Pro Ala Thr Tyr Thr Val Asp Ala Gln Thr Gly Lys Met Ala
   220                 225                 230                 235
   cat ctt gat gtc tgg gac tct tgg cca gta caa gat cct gtc aca ggt    1972
   His Leu Asp Val Trp Asp Ser Trp Pro Val Gln Asp Pro Val Thr Gly
                   240                 245                 250
   tat gta tct aat tac atg ggt tat caa cta gtt att gct atg atg ggt    2020
   Tyr Val Ser Asn Tyr Met Gly Tyr Gln Leu Val Ile Ala Met Met Gly
               255                 260                 265
   att cca aat tcg cca act gga gat aat cat atc tat ctt ctt tac aac    2068
   Ile Pro Asn Ser Pro Thr Gly Asp Asn His Ile Tyr Leu Leu Tyr Asn
           270                 275                 280
   aag tat ggt gat aat gac ttt tct cat tgg cgc aat gca ggt tca atc    2116
   Lys Tyr Gly Asp Asn Asp Phe Ser His Trp Arg Asn Ala Gly Ser Ile
       285                 290                 295
   ttt gga act aaa gaa aca aat gtg ttc caa gaa tgg tca ggt tca gct    2164
   Phe Gly Thr Lys Glu Thr Asn Val Phe Gln Glu Trp Ser Gly Ser Ala
   300                 305                 310                 315
   att gta aat gat gat ggt aca att caa cta ttt ttc acc tca aat gat    2212
   Ile Val Asn Asp Asp Gly Thr Ile Gln Leu Phe Phe Thr Ser Asn Asp
                   320                 325                 330
   acg tct gat tac aag ttg aat gat caa cgc ctt gct acc gca aca tta    2260
   Thr Ser Asp Tyr Lys Leu Asn Asp Gln Arg Leu Ala Thr Ala Thr Leu
               335                 340                 345
   aac ctt aat gtt gat gat aac ggt gtt tca atc aag agt gtt gat aat    2308
   Asn Leu Asn Val Asp Asp Asn Gly Val Ser Ile Lys Ser Val Asp Asn
           350                 355                 360
   tat caa gtt ttg ttt gaa ggt gat gga ttt cac tac caa act tat gaa    2356
   Tyr Gln Val Leu Phe Glu Gly Asp Gly Phe His Tyr Gln Thr Tyr Glu
       365                 370                 375
   caa ttc gca aac ggc aaa gat cgt gaa aat gat gat tac tgc tta cgt    2404
   Gln Phe Ala Asn Gly Lys Asp Arg Glu Asn Asp Asp Tyr Cys Leu Arg
   380                 385                 390                 395
   gac cca cac gtt gtt caa tta gaa aat ggt gat cgt tat ctt gta ttc    2452
   Asp Pro His Val Val Gln Leu Glu Asn Gly Asp Arg Tyr Leu Val Phe
                   400                 405                 410
   gaa gct aat act ggg aca gaa gat tac caa agt gac gac caa att tat    2500
   Glu Ala Asn Thr Gly Thr Glu Asp Tyr Gln Ser Asp Asp Gln Ile Tyr
               415                 420                 425
   aat tgg gct aac tat ggt ggc gat gat gcc ttc aat att aag agt tcc    2548
   Asn Trp Ala Asn Tyr Gly Gly Asp Asp Ala Phe Asn Ile Lys Ser Ser
           430                 435                 440
   ttc aag ctt ttg aat aat aag aag gat cgt gaa ttg gct ggt tta gct     2596
   Phe Lys Leu Leu Asn Asn Lys Lys Asp Arg Glu Leu Ala Gly Leu Ala
       445                 450                 455
   aat ggt gca ctt ggt atc tta aag ctc act aac aat caa agt aag cca     2644
   Asn Gly Ala Leu Gly Ile Leu Lys Leu Thr Asn Asn Gln Ser Lys Pro
   460                 465                 470                 475
   aag gtt gaa gaa gta tac tca cca ttg gta tct act ttg atg gct tgc     2692
   Lys Val Glu Glu Val Tyr Ser Pro Leu Val Ser Thr Leu Met Ala Cys
                   480                 485                 490
   gat gag gta nnn nnn aag ctt ggt gat aag tat tat ctc ttc tcc gta     2740
   Asp Glu Val Xaa Xaa Lys Leu Gly Asp Lys Tyr Tyr Leu Phe Ser Val
               495                 500                 505
   act cgt gta agt cgt ggt tcc gat cgt gaa tta acc gct aag gat aac     2788
   Thr Arg Val Ser Arg Gly Ser Asp Arg Glu Leu Thr Ala Lys Asp Asn
           510                 515                 520
   aca atc gtt ggt gat aac gtt gct atg att ggt tac gtt tcc gat agc     2836
   Thr Ile Val Gly Asp Asn Val Ala Met Ile Gly Tyr Val Ser Asp Ser
       525                 530                 535
   tta atg ggt aag tac aag cca tta aat aac tca ggt gtc gta tta act     2884
   Leu Met Gly Lys Tyr Lys Pro Leu Asn Asn Ser Gly Val Val Leu Thr
   540                 545                 550                 555
   gca tca gta cct gca aac tgg cgt act gct act tat tcc tac tat gca     2932
   Ala Ser Val Pro Ala Asn Trp Arg Thr Ala Thr Tyr Ser Tyr Tyr Ala
                   560                 565                 570
   gta cct gta gct ggt cat cct gat caa gta tta att act tct tac atg     2980
   Val Pro Val Ala Gly His Pro Asp Gln Val Leu Ile Thr Ser Tyr Met
               575                 580                 585
   agt aac aag gac ttt gct tca ggt gaa gga aac tat gca act tgg gca     3028
   Ser Asn Lys Asp Phe Ala Ser Gly Glu Gly Asn Tyr Ala Thr Trp Ala
           590                 595                 600
   cca agt ttc tta gta caa atc aat cca gat gac acg aca act gta tta     3076
   Pro Ser Phe Leu Val Gln Ile Asn Pro Asp Asp Thr Thr Thr Val Leu
       605                 610                 615
   gca cgt gca act aac caa ggt gac tgg gtg tgg gac gac tct agt cgg     3124
   Ala Arg Ala Thr Asn Gln Gly Asp Trp Val Trp Asp Asp Ser Ser Arg
   620                 625                 630                 635
   aac gat aat atg ctc ggt gtt ctt aaa gaa ggt gca gct aac agt gcc    3172
   Asn Asp Asn Met Leu Gly Val Leu Lys Glu Gly Ala Ala Asn Ser Ala
                   640                 645                 650
   gcc tta cca ggt gaa tgg ggt aag cca gtt gac tgg agt ttg att aac    3220
   Ala Leu Pro Gly Glu Trp Gly Lys Pro Val Asp Trp Ser Leu Ile Asn
               655                 660                 665
   aga agt cct ggc tta ggc tta aag cct cat caa cca gtt caa cca aag    3268
   Arg Ser Pro Gly Leu Gly Leu Lys Pro His Gln Pro Val Gln Pro Lys
           670                 675                 680
   att gat caa cct gat caa caa cct tct ggt caa aac act aag aat gtc    3316
   Ile Asp Gln Pro Asp Gln Gln Pro Ser Gly Gln Asn Thr Lys Asn Val
       685                 690                 695
   aca cca ggt aat ggt gat aag cct gct ggt aag gca act cct gat aac    3364
   Thr Pro Gly Asn Gly Asp Lys Pro Ala Gly Lys Ala Thr Pro Asp Asn
   700                 705                 710                 715
   act aat att gat cca agt gca caa cct tct ggt caa aac act aat att    3412
   Thr Asn Ile Asp Pro Ser Ala Gln Pro Ser Gly Gln Asn Thr Asn Ile
                   720                 725                 730
   gat cca agt gca caa mct tct ggt caa aac act aag aat gtc aca cca    3460
   Asp Pro Ser Ala Gln Xaa Ser Gly Gln Asn Thr Lys Asn Val Thr Pro
               735                 740                 745
   ggt aat gag aaa caa ggt aag aat acc gat gca aaa caa tta cca caa    3508
   Gly Asn Glu Lys Gln Gly Lys Asn Thr Asp Ala Lys Gln Leu Pro Gln
           750                 755                 760
   aca ggt aat aag tct ggt tta gca gga ctt tac gct ggt tca tta ctt    3556
   Thr Gly Asn Lys Ser Gly Leu Ala Gly Leu Tyr Ala Gly Ser Leu Leu
       765                 770                 775
   gcc ttg ttt gga ttg gca gca att gaa aag cgt cac gct taa            3598
   Ala Leu Phe Gly Leu Ala Ala Ile Glu Lys Arg His Ala
   780                 785                 790
   tagagtaaaa aaacatcctc cactcaagtt acaagtagga taatatgtat tatttctacg 3658
   cytagtcaag aggrattact ggacatannn nnnnnnnnnn tccagttacc aagtggaata 3718
   tagtattatt ccacgctagt caggaggatt actgacatta ttggctacat ggccggtagt 3778
   cctcttttct tttgtgacga attgtcaaac caagtgcaac ggtttctcaa aaaacacctc 3838
   atatggggtt tcataattta acacttttcg aggacggcgg ttcagctgat gttggcagaa 3898
   actgacgtcc ttatctgtat aatcatcaat attagccctt ttaggaaagt attccctaat 3958
   tagsccattg gtattttcat tgggtcctct ttcctctggt gaatagggat ctggccaata 4018
   gatagctact cctaaacgtc ctcgaatatc attcaagcca agaaattcac gcccatgatc 4078
   tggagtcaat gaatggacaa attctttagg aatagaccct aagagatcaa ttaagccctg 4138
   atatttgaat tcggagaagg ggagttgtcc aacaattgcc gttataatac cagggttaat 4198
   acggccctgg gcctctacgg taatattgta tttttggctc agatcagtga tagaaaccca 4258
   cagatttagc ttgccggtgg agtgctgctt gaagtcttca attacttcgt taccatgttt 4318
   gattgctaat ctgatgtgtc gttgttgtgg tgtagtaggc atcataccac ctcctcataa 4378
   aataaggtat aacaggaatt tcttgtacta tatgatcctt ccaatataat aatattaggc 4438
   cgataagaaa tgaccagcta ccatttcttg atgcttagtg aatataatcg gatgatacgt 4498
   cacccctcaa caatccaatt tcacggaggt gagtaatcat gccgagagct aggaatgatt 4558
   ggaggaacga acacggtcca tgcggcagtg gctatttgga ttttagccaa agcagcgtta 4618
   ctgcttgcaa aagctt 4634
<210> 11
   <211> 792
   <212> PRT
   <213> Lactobacillus reuteri
<400> 11
   Met Tyr Lys Val Gly Lys Asn Trp Ala Val Ala Thr Leu Val Ser Ala
   1 5 10 15
   Ser Ile Leu Met Gly Gly Val Val Thr Ala His Ala Asp Gln Val Glu
   20 25 30
   Ser Asn Asn Tyr Asn Gly Val Ala Glu Val Asn Thr Glu Arg Gln Ala
   35 40 45
   Asn Gly Gln Ile Gly Val Asp Gly Lys Ile Ile Ser Ala Asn Ser Asn
           50                  55                  60
   Thr Thr Ser Gly Ser Thr Asn Gln Glu Ser Ser Ala Thr Asn Asn Thr
    65                  70                  75                  80
   Glu Asn Ala Val Val Asn Glu Ser Lys Asn Thr Asn Asn Thr Glu Asn
                    85                  90                  95
   Ala Val Val Asn Glu Asn Lys Asn Thr Asn Asn Thr Glu Asn Ala Val
               100                 105                 110
   Val Asn Glu Asn Lys Asn Thr Asn Asn Thr Glu Asn Asp Asn Ser Gln
           115                 120                 125
   Leu Lys Leu Thr Asn Asn Glu Gln Pro Ser Ala Ala Thr Gln Ala Asn
       130                 135                 140
   Leu Lys Lys Leu Asn Pro Gln Ala Ala Lys Ala Val Gln Asn Ala Lys
   145                 150                 155                 160
   Ile Asp Ala Gly Ser Leu Thr Asp Asp Gln Ile Asn Glu Leu Asn Lys
                   165                 170                 175
   Ile Asn Phe Ser Lys Ser Ala Glu Lys Gly Ala Lys Leu Thr Phe Lys
               180                 185                 190
   Asp Leu Glu Gly Ile Gly Asn Ala Ile Val Lys Gln Asp Pro Gln Tyr
           195                 200                 205
   Ala Ile Pro Tyr Ser Asn Ala Lys Glu Ile Lys Asn Met Pro Ala Thr
       210                 215                 220
   Tyr Thr Val Asp Ala Gln Thr Gly Lys Met Ala His Leu Asp Val Trp
   225                 230                 235                 240
   Asp Ser Trp Pro Val Gln Asp Pro Val Thr Gly Tyr Val Ser Asn Tyr
                   245                 250                 255
   Met Gly Tyr Gln Leu Val Ile Ala Met Met Gly Ile Pro Asn Ser Pro
               260                 265                 270
   Thr Gly Asp Asn His Ile Tyr Leu Leu Tyr Asn Lys Tyr Gly Asp Asn
           275                 280                 285
   Asp Phe Ser His Trp Arg Asn Ala Gly Ser Ile Phe Gly Thr Lys Glu
       290                 295                 300
   Thr Asn Val Phe Gln Glu Trp Ser Gly Ser Ala Ile Val Asn Asp Asp
   305 310 315 320
   Gly Thr Ile Gln Leu Phe Phe Thr Ser Asn Asp Thr Ser Asp Tyr Lys
   325 330 335
   Leu Asn Asp Gln Arg Leu Ala Thr Ala Thr Leu Asn Leu Asn Val Asp
   340 345 350
   Asp Asn Gly Val Ser Ile Lys Ser Val Asp Asn Tyr Gln Val Leu Phe
   355 360 365
   Glu Gly Asp Gly Phe His Tyr Gln Thr Tyr Glu Gln Phe Ala Asn Gly
   370 375 380
   Lys Asp Arg Glu Asn Asp Asp Tyr Cys Leu Arg Asp Pro His Val Val
   385 390 395 400
   Gln Leu Glu Asn Gly Asp Arg Tyr Leu Val Phe Glu Ala Asn Thr Gly
   405 410 415
   Thr Glu Asp Tyr Gln Ser Asp Asp Gln Ile Tyr Asn Trp Ala Asn Tyr
   420 425 430
   Gly Gly Asp Asp Ala Phe Asn Ile Lys Ser Ser Phe Lys Leu Leu Asn
   435 440 445
   Asn Lys Lys Asp Arg Glu Leu Ala Gly Leu Ala Asn Gly Ala Leu Gly
   450 455 460
   Ile Leu Lys Leu Thr Asn Asn Gln Ser Lys Pro Lys Val Glu Glu Val
   465 470 475 480
   Tyr Ser Pro Leu Val Ser Thr Leu Met Ala Cys Asp Glu Val Xaa Xaa
   485 490 495
   Lys Leu Gly Asp Lys Tyr Tyr Leu Phe Ser Val Thr Arg Val Ser Arg
   500 505 510
   Gly Ser Asp Arg Glu Leu Thr Ala Lys Asp Asn Thr Ile Val Gly Asp
   515 520 525
   Asn Val Ala Met Ile Gly Tyr Val Ser Asp Ser Leu Met Gly Lys Tyr
   530 535 540
   Lys Pro Leu Asn Asn Ser Gly Val Val Leu Thr Ala Ser Val Pro Ala
   545 550 555 560
   Asn Trp Arg Thr Ala Thr Tyr Ser Tyr Tyr Ala Val Pro Val Ala Gly
   565 570 575
   His Pro Asp Gln Val Leu Ile Thr Ser Tyr Met Ser Asn Lys Asp Phe
   580 585 590
   Ala Ser Gly Glu Gly Asn Tyr Ala Thr Trp Ala Pro Ser Phe Leu Val
   595 600 605
   Gln Ile Asn Pro Asp Asp Thr Thr Thr Val Leu Ala Arg Ala Thr Asn
   610 615 620
   Gln Gly Asp Trp Val Trp Asp Asp Ser Ser Arg Asn Asp Asn Met Leu
   625 630 635 640
   Gly Val Leu Lys Glu Gly Ala Ala Asn Ser Ala Ala Leu Pro Gly Glu
   645 650 655
   Trp Gly Lys Pro Val Asp Trp Ser Leu Ile Asn Arg Ser Pro Gly Leu
   660 665 670
   Gly Leu Lys Pro His Gln Pro Val Gln Pro Lys Ile Asp Gln Pro Asp
   675 680 685
   Gln Gln Pro Ser Gly Gln Asn Thr Lys Asn Val Thr Pro Gly Asn Gly
   690 695 700
   Asp Lys Pro Ala Gly Lys Ala Thr Pro Asp Asn Thr Asn Ile Asp Pro
   705 710 715 720
   Ser Ala Gln Pro Ser Gly Gln Asn Thr Asn Ile Asp Pro Ser Ala Gln
   725 730 735
   Xaa Ser Gly Gln Asn Thr Lys Asn Val Thr Pro Gly Asn Glu Lys Gln
   740 745 750
   Gly Lys Asn Thr Asp Ala Lys Gln Leu Pro Gln Thr Gly Asn Lys Ser
   755 760 765
   Gly Leu Ala Gly Leu Tyr Ala Gly Ser Leu Leu Ala Leu Phe Gly Leu
   770 775 780
   Ala Ala Ile Glu Lys Arg His Ala
   785 790
<210> 12
   <211> 24
   <212> DNA
   <213> Artificial Sequence
<220>
   <223> Description of Artificial Sequence:primer
<400> 12
   ctgataataa tggaaatgta tcac 24
<210> 13
   <211> 26
   <212> DNA
   <213> Artificial Sequence
<220>
   <223> Description of Artificial Sequence:primer
<400> 13
   catgatcata agtttggtag taatag 26
<210> 14
   <211> 24
   <212> DNA
   <213> Artificial Sequence
<220>
   <223> Description of Artificial Sequence:primer
<400> 14
   gtgatacatt tccattatta tcag 24
<210> 15
   <211> 26
   <212> DNA
   <213> Artificial Sequence
<220>
   <223> Description of Artificial Sequence:primer
<400> 15
   ctattactac caaacttatg atcatg 26
<210> 16
   <211> 38
   <212> DNA
   <213> Artificial Sequence
<220>
   <223> Description of Artificial Sequence:primer
<400> 16
   ccatggccat ggtagaacgc aaggaacata aaaaaatg 38
<210> 17
   <211> 38
   <212> DNA
   <213> Artificial Sequence
<220>
   <223> Description of Artificial Sequence:primer
<400> 17
   agatctagat ctgttaaatc gacgtttgtt aatttctg 38
<210> 18
   <211> 21
   <212> DNA
   <213> Artificial Sequence
<220>
   <223> Description of Artificial Sequence:primer
<400> 18
   gaygtntggg aywsntgggc c 21
<210> 19
   <211> 23
   <212> DNA
   <213> Artificial Sequence
<220>
   <223> Description of Artificial Sequence:primer
<400> 19
   gtngcnswnc cnswccayts ytg 23
<210> 20
   <211> 22
   <212> DNA
   <213> Artificial Sequence
<220>
   <223> Description of Artificial Sequence:primer
<400> 20
   gaatgtaggt ccaatttttg gc 22
<210> 21
   <211> 22
   <212> DNA
   <213> Artificial Sequence
<220>
   <223> Description of Artificial Sequence:primer
<400> 21
   cctgtccgaa catcttgaac tg 22
<210> 22
   <211> 23
   <212> DNA
   <213> Artificial Sequence
<220>
   <223> Description of Artificial Sequence:primer
<400> 22
   arraanswng gngcvmangt nsw 23
<210> 23
   <211> 23
   <212> DNA
   <213> Artificial Sequence
<220>
   <223> Description of Artificial Sequence:primer
<400> 23
   tayaayggng tngcngargt naa 23
<210> 24
   <211> 22
   <212> DNA
   <213> Artificial Sequence
<220>
   <223> Description of Artificial Sequence:primer
<400> 24
   ccgaccatct tgtttgatta ac 22
<210> 25
   <211> 24
   <212> DNA
   <213> Artificial Sequence
<220>
   <223> Description of Artificial Sequence:primer
<400> 25
   aaytataayg gygttgcryg aagt 24
<210> 26
   <211> 21
   <212> DNA
   <213> Artificial Sequence
<220>
   <223> Description of Artificial Sequence:primer
<400> 26
   taccgnwsnc tacttcaact t 21